# EUROPEAN PATENT SPECIFICATION

(11) **EP 3 559 027 B1**
(45) Date of publication and mention of the grant of the patent: **01.06.2022**
(21) Application number: 17835870.1
(22) Date of filing: 20.12.2017
(51) Int. Cl.: C07K 16/00, C07K 16/10, A61K 39/395, A61P 31/14

(54) **METHODS FOR ENHANCING ANTIBODY PRODUCTIVITY IN MAMMALIAN CELL CULTURE AND MINIMIZING AGGREGATION DURING DOWNSTREAM, FORMULATION PROCESSES AND STABLE ANTIBODY FORMULATIONS OBTAINED THEREOF**
VERFAHREN ZUR ERHÖHUNG DER ANTIKÖRPERPRODUKTIVITÄT IN SÄUGETIERZELLKULTUR UND ZUR MINIMIERUNG DER AGGREGATION BEIM DOWNSTREAM, FORMULIERUNGSVERFAHREN UND DARAUS ERHALTENE STABILE ANTIKÖRPERFORMULIERUNGEN
PROCÉDÉS POUR AUGMENTER LA PRODUCTIVITÉ D'ANTICORPS DANS UNE CULTURE DE CELLULES DE MAMMIFÈRE ET MINIMISER L'AGRÉGATION PENDANT DES PROCESSUS DE FORMULATION EN AVAL, PROCÉDÉS DE FORMULATION ET FORMULATIONS D'ANTICORPS STABLES OBTENUS À PARTIR DE CEUX-CI

(30) Priority: 23.12.2016 IN 201621044139
(43) Date of publication of application: 30.10.2019
(73) Proprietor: Serum Institute Of India Private Limited, Pune, Maharashtra 411 028 (IN)
(72) Inventor: MHALASAKANT, Dhere Rajeev, Pune 411 028 Maharashtra (IN); SHANKAR, Pisal Sambhaji, Pune 411 028 Maharashtra (IN); REDDY, Peddi Reddy Srinivas, Pune 411 028 Maharashtra (IN); CHAHAR, Singh Digamber, Pune 411 028 Maharashtra (IN); RAVINDRA, Yeolekar Leena, Pune 411 052 (IN); SINGH, Chouhan Pankaj, Pune 411 028 Maharashtra (IN); DATTATRAY, Avalaskar Nikhil, Pune 411 028 Maharashtra (IN)
(74) Representative: Ström & Gulliksson AB
(86) International application number: PCT/IB2017/058194
(87) International publication number: WO 2018/116198

(56) References cited:
- WO-A1-2006/084006
- WO-A1-2012/125735
- WO-A1-2015/122995
- WO-A1-2017/165736
- WO-A2-2011/085158
- WO-A2-2011/104381
- WO-A2-2012/151247
- US-A1- 2017 088 873
- US-B1- 8 613 919
- KUNERT RENATE ET AL: "Advances in recombinant antibody manufacturing", APPLIED MICROBIOLOGY AND BIOTECHNOLOGY, SPRINGER, DE, vol. 100, no. 8, 3 March 2016 (2016-03-03) , pages 3451-3461, XP035870818, ISSN: 0175-7598, DOI: 10.1007/S00253-016-7388-9 [retrieved on 2016-03-03]
- REINHART DAVID ET AL: "Benchmarking of commercially available CHO cell culture media for antibody production", APPLIED MICROBIOLOGY AND BIOTECHNOLOGY, SPRINGER, DE, vol. 99, no. 11, 7 April 2015 (2015-04-07) , pages 4645-4657, XP035502970, ISSN: 0175-7598, DOI: 10.1007/S00253-015-6514-4 [retrieved on 2015-04-07]
- LIANCHUN FAN ET AL: "Improving the efficiency of CHO cell line generation using glutamine synthetase gene knockout cells", BIOTECHNOLOGY AND BIOENGINEERING, vol. 109, no. 4, 21 November 2011 (2011-11-21), pages 1007-1015, XP055213290, ISSN: 0006-3592, DOI: 10.1002/bit.24365
- PETRA GRONEMEYER ET AL: "Trends in upstream and downstream process development for antibody manufacturing", BIOENGINEERING (BASEL, SWITZERLAND) 08 MAY 2017, vol. 1, no. 4, 1 October 2014 (2014-10-01) , pages 188-212, XP055461223, ISSN: 2306-5354, DOI: 10.3390/bioengineering1040188
- SHUKLA A A ET AL: "Recent advances in large-scale production of monoclonal antibodies and related proteins", TRENDS IN BIOTECHNOLOGY, ELSEVIER PUBLICATIONS, CAMBRIDGE, GB, vol. 28, no. 5, 1 May 2010 (2010-05-01), pages 253-261, XP027020088, ISSN: 0167-7799 [retrieved on 2010-03-19]
- DAUGHERTY ANN L ET AL: "CHAPTER 8: Formulation and delivery issues for monoclonal antibody therapeutics", CURRENT TRENDS IN MONOCLONAL ANTIBODY DEVELOPMENT AND MANUFACTU, SPRINGER, US, 1 January 2010 (2010-01-01), pages 103-129, XP009180430, ISBN: 978-0-387-76642-3
- Anonymous: "Scale-up of CHO fed-batch cultures in HyClone(TM) ActiPro(TM) medium supplemented with Cell Boost(TM) 7a and 7b", , 11 October 2016 (2016-10-11), pages 1-4, XP055461185, Hoboken Retrieved from the Internet: URL:http://www.processdevelopmentforum.com /ppts/posters/Scale-up_of_CHO_fed-batch_cu ltures_in_HyClone(TM)_ActiPro.PDF [retrieved on 2018-03-20]
- SOMMERFELD S ET AL: "Challenges in biotechnology production-generic processes and process optimization for monoclonal antibodies", CHEMICAL ENGINEERING AND PROCESSING, ELSEVIER SEQUOIA, LAUSANNE, CH, vol. 44, no. 10, 1 October 2005 (2005-10-01), pages 1123-1137, XP027608266, ISSN: 0255-2701 [retrieved on 2005-10-01] cited in the application

## Description

### Background of the Invention

Upstream, Downstream and formulation development can often be the rate-limiting step in the early introduction of biopharmaceuticals into clinical trials. For instance, Dengue is the most important mosquito-borne viral disease affecting humans. Half of the world population lives in areas at risk for dengue, resulting in an estimated 390 million infections per year globally. Currently no antiviral agents are approved for treating dengue and recent vaccine trials have fallen short of expectations. The leading vaccine candidate recently demonstrated limited efficacy, estimated to be between 30%- 60%, with limited to no significant protection against DENV-2. Recently a non-immunodominant, but functionally relevant, epitope in domain III of the E protein has been identified, and subsequently an engineered antibody, Ab513 is being developed that exhibits high-affinity binding to, and broadly neutralizes, multiple genotypes within all four serotypes (Refer Ram Sasisekharan et al Cell 162, 1-12, July 30, 2015; Samir Bhatt et al, Nature, 2013 April 25; 496 7446: 504-507). Thus, if we consider global medical demand for a Dengue monoclonal antibody, recent estimates indicate that up to 390 million dengue infections occur every year globally with >90 million presenting with disease making DENV a major global threat. If we assume that 30% of the 16 million diagnosed dengue cases go to hospital then about 5 million will require said Dengue monoclonal antibody, which implies that "purified antibody" above 4 gm/L becomes a prerequisite to meet global demand of such a life saving antibody. Further, Dengue disease burden is high in developing countries where availability of electrical power and refrigeration are often inadequate and therefore antibody stability across temperature excursions assumes greater relevance for these regions.

Indeed, if it was possible to have a platform process that could be employed for manufacturing and formulating all monoclonal antibody (mAb) candidates it would greatly reduce the time and resources needed for process development. This can have a significant impact on the number of clinical candidates that can be introduced into clinical trials. Also, processes developed for early stage clinical trials, including those developed using a platform, may be non-optimal with respect to process economics, yield, pool volumes, throughput and may not be suitable for producing the quantities required for late stage or commercial campaigns. Another important consideration is the speed of process development given that process development needs to occur prior to introduction of a therapeutic candidate into clinical trials. (Refer Abhinav A. Shukla et al Journal of Chromatography B, 848 (2007) 28-39).

Typically, mammalian cell culture media is based on commercially available media formulations, including, for example, DMEM or Ham's F12. Often media formulations are not sufficiently enriched to support increases in both cell growth and biologic protein expression. There remains a need for improved cell culture media, supplements, and cell culture methods for improved protein production. Increases in cell culture antibody titers to >2 g/L have been reported earlier. Refer F. Wurm, Nat. Biotechnol. 22 (2004) 1393. Further, in perfusion reactors, cells can reach much higher cell densities than in conventional batch or fed-batch reactors. (Refer Sven Sommerfeld et al Chemical Engineering and Processing 44 (2005) 1123-1137). However, perfusion based processes are complex, costly and may also result in sterility issues and undesired heterogeneity in glycosylation pattern. Addition of animal-component-free hydrolysates (Bacto TC Yeastolate, Phytone Peptone) to chemically defined media is a common approach to increase cell density, culture viability and productivity in a timely manner. Hydrolysates are protein digests composed of amino acids, small peptides, carbohydrates, vitamins and minerals that provide nutrient supplements to the media. Nonanimal derived hydrolysates from soy, wheat and yeast are used commonly in cell culture media and feeds to improve antibody titer (Refer US9284371). However, because of its composition complexity, lot-to-lot variations, undesirable attribute of making culture viscous, Yeast extract and hydrolysates can be a significant source of medium variability. Due to the complexity of antibody products that include isoforms and micro heterogeneities, the performance of the cell culture process can have significant effects on product quality and potency, especially with respect to glycosylation, post-transcriptional modifications and impurity profiles.

At higher concentrations, proteins, particularly antibodies often exhibit characteristic problems including aggregation, precipitation, gelation, lowered stability, and/or increased viscosity.

Antibodies are recognized as possessing characteristics that tend to form aggregates and particulates in solution as they undergo degradation or aggregation or denaturation or chemical modifications resulting in the loss of biological activity during the manufacturing process and / or during storage with time. Antibody aggregates could be formed during cell culture expression, downstream purification, formulation and on storage. Cell culture harvest usually contains the highest level of aggregate in the process (Refer Deqiang Yu Journal of Chromatography A, 1457 (2016) 66-75). Degradation pathways for proteins can involve chemical instability (e.g., any process which involves modification of the protein by bond formation or cleavage resulting in a new chemical entity) or physical instability (e.g., changes in the higher order structure of the protein). The three most common protein degradation pathways are protein aggregation, deamidation and oxidation. Cleland et al Critical Reviews in Therapeutic Drug Carrier Systems 10(4): 307-377 (1993). Further, proteins also are sensitive to, for example, pH, ionic strength, thermal stress, shear and interfacial stresses, all of which can lead to aggregation and result in instability. For a protein to remain biologically active, a formulation must therefore preserve intact the conformational integrity of at least a core sequence of the protein's amino acids while at the same time protecting the protein's multiple functional groups from degradation.

A major problem caused by the aggregate formation is that during the administration the formulation may block syringes or pumps and rendering it unsafe to patients. Such protein modifications can also make them immunogenic resulting in the generation of anti-drug antibodies by the patient which can reduce the drug availability during subsequent injections or worse induce an autoimmune reaction. A major aim in the development of antibody formulations is to maintain protein solubility, stability and bioactivity.

Early suggestions about how to solve the problems of instability of protein therapeutics formulations included the lyophilization of the drug product, followed by reconstitution immediately or shortly prior to administration. However, Lyophilized formulations of antibodies have a number of limitations, including a prolonged process for lyophilization and resulting high cost for manufacturing. In addition, a lyophilized formulation has to be reconstituted aseptically and accurately by healthcare practitioners prior to administering to patients. The reconstitution step itself requires certain specific procedures, i.e. (1) a sterile diluent (i.e., water for intravenous administration and 5% dextrose in water for intramuscular administration) is added to the vial containing lyophilized antibody, slowly and aseptically, and the vial must be swirled very gently for 30 seconds to avoid foaming; (2) the reconstituted antibody may need to stand at room temperature for a minimum of 20 minutes until the solution clarifies; and (3) the reconstituted preparation must be administered within six (6) hours after the reconstitution. Such reconstitution procedure is cumbersome and the time limitation after the reconstitution can cause a great inconvenience in administering the formulation to patients, leading to significant waste, if not reconstituted properly, or if the reconstituted dose is not used within six (6) hours and must be discarded. Therefore, a liquid formulation is desirable due to factors of clinical and patient convenience as well as ease of manufacture. However liquid pharmaceutical formulations of protein therapeutics, i.e. antibodies should be long-term stable, contain a safe and effective amount of the pharmaceutical compound.

Removal of aggregates is more difficult than removal of process related impurities due to the biophysical similarities between the aggregate and monomer, the multiple sources and types of aggregate, and less understanding of aggregation mechanism.

One of the more recent challenges encountered during formulation development of high concentration monoclonal antibody dosage forms is the formation of proteinaceous subvisible and visible particulates during manufacturing and long-term storage. The level of proteinaceous and non-proteinaceous particulates in IgG formulations is an increasingly important part of purification and formulation development. (Refer Klaus Wuchner et al Journal of Pharmaceutical Sciences, vol. 99, no. 8, august 2010). Further the liquid formulation should be stable across different temperatures viz temperatures 2-8° C, 25° C, 40° C , and 55° C.

Many antibody preparations intended for human use require stabilizers to prevent denaturation, aggregation and other alternations to the proteins prior to the use of the preparation. Previously reported antibody Liquid antibody formulations (Lucentis, Avastin) had mannitol, trehalose as stabilizers. (Refer Susumu Uchiyama et al Biochimica Biophysica Acta 1844 (2014) 2041-2052; US20160137727; WO2009120684; US8568720). However trehalose is costly and not feasible from large scale process economics.

Also, the IV administration of antibody is usually given as an infusion rather than a bolus, and thus requires dilution of mAb formulation, including excipients into appropriate fluids suitable for IV administration. The resulting dilution of excipients, especially surfactants, which may decrease below the concentration required for prevention of aggregation during agitation, thereby resulting in generation of aggregates and subvisible particles following gentle agitation after dilution into PVC and PO IV bags containing 0.9% saline.

Hydrophobic interaction chromatography, ceramic hydroxyapatite and cation exchange resins have all been used for aggregate removal but none are ideal. Majority of previously reported antibody purification processes have heavily relied upon use of Hydrophobic interaction chromatography in combination with Protein A chromatography, Anion exchange chromatography, Cation exchange chromatography as a three or four step process (Refer WO2010141039 , WO 2014/207763, WO2013066707, WO2015099165, WO2014102814, WO2015038888, WO2004087761). However, Hydrophobic interaction chromatography resins require large amounts of salts that are expensive, show low binding capacity, can be difficult to dispose of, and may not be compatible with the materials of construction of buffer and product holding tanks. Furthermore, the density difference between the buffers used for a HIC step can cause bed stability problems. Ceramic hydroxyapatite can also be used for the separation of aggregate from monomer, but the ceramic resin can be very difficult to unpack without damaging the resin. Therefore, storing the resin outside the column for re-use in a subsequent manufacturing campaign may not be possible (Refer Suzanne Aldington Journal of Chromatography B, 848 (2007) 64-78).

Three-step combinations of cation-exchange, anion-exchange flow through, hydrophobic interaction chromatography and mixed mode cation-exchange chromatography were found to deliver adequate clearance of host cell protein contaminants for a CHO derived monoclonal antibody. However, such purification schemes by-and-large have not caught on in commercial downstream operations due to the need to design the purification sequence separately for each mAb.

WO 2015/122995 A1 discloses an anti-Dengue virus antibody D88 comprising the V_{H} and V_{L} domains. WO 2006/084006 A1 discloses anti-rabies virus antibody 17C7 as well as production thereof. WO 2017/165736 A1 discloses various formulations of anti-Dengue antibodies.

Thus, there is an urgent unmet need for an efficient platform process for antibody manufacturing and formulation that meets multiple criterion including robustness, reliability and scalability, in particular a platform that provides i) antibody titer of at least 2 gm/L; ii) minimum aggregation/particulate formation across cell culture, purification and formulation processes; iii) improved purification showing optimal percentage recovery, high monomer content and minimum impurity levels; and iv) high concentration antibody formulation showing low viscosity, devoid of aggregation and sub-visible particles; thereby showing long-term stability.

### Summary

### Applicant has surprisingly found

a) A feed composition and feeding strategy that takes into consideration nutrient consumption, by-product accumulation and the balance between promoting growth versus volumetric productivity wherein in particular, mammalian cell culture process parameters like use of particular basal medium, use of concentrated basal medium as feed solution, use of different feed solutions along-with a definite feeding strategy, maintaining lower concentrations of lactate and ammonia, are found to enhance cell growth, cell longevity and protein expression; thereby resulting in an increased antibody titer.
b) Specific salt concentration as part of buffer during Protein A affinity and Cation exchange steps that minimizes aggregation; thereby achieving a monomer content of greater than 99% with a recovery of greater than 80%.
c) Particle free liquid antibody formulations comprising of sucrose in combination with Histidine, Arginine, Polysorbate-80, Sodium chloride that impart higher potency and stability, reduces viscosity of highly concentrated antibody solutions at 2-8°C for at least 9 months, at 25° C for at least 1 month, at 40° C for at least 42 days, at 55° C for at least 2 days as compared to formulation devoid of sucrose.

A first aspect of the present disclosure relates to a method of manufacturing pharmaceutical antigen binding protein with high yield and minimum aggregation, wherein the antigen binding protein is an anti-Dengue monoclonal antibody according to SEQ ID 1 and SEQ ID 2 or wherein the antigen binding protein is an anti-Rabies monoclonal antibody according to SEQ ID 3 and SEQ ID 4, the method comprising the steps of: a) culturing in large scale mammalian cells that express antigen binding protein in a cell culture production medium, wherein the culturing step effectively maintains the cell count in the range of 10 x 10⁶ - 20 x 10⁶ cells/ml and results in a yield of at least 2 gm/L; wherein the cell line of mammalian cells is CHO-K1 SV GS-KO when the antigen binding protein is an anti-Dengue monoclonal antibody and the cell line of mammalian cells is GS-CHO when the antigen binding protein is an anti-Rabies monoclonal antibody; wherein the culturing step includes use of basal medium, use of concentrated basal medium as feed solution, use of feed solutions along-with a definite feeding strategy, resulting in enhanced cell growth, maintaining lower concentrations of lactate and ammonia, and effectively maintaining the cell count thereby increasing cell longevity and high yield; b) purification of antigen binding protein from harvested supernatant obtained in step (a), wherein the purification results in recovery of at least 80% and purity of at least 99%; and wherein the purification comprises affinity chromatography, Low pH viral inactivation, cation exchange chromatography, anion exchange chromatography, nanofiltration, Tangential flow filtration/Ultrafiltration; in a sequential manner; wherein the affinity chromatography matrix is Protein A; wherein the salt concentration of the buffers used in purification is in the range of 30 mM - 500 mM; and c) preparing a stable formulation comprising the antigen binding protein, wherein osmolality of the formulation is in range of 300 - 400 mOsm/Kg and the viscosity of the formulation is less than 2.5 mPa-S, wherein the formulation comprises at least one antigen binding protein, at least one stabilizer, at least one buffering agent, at least one tonicity agent, and at least one surfactant; wherein the formulation comprises 1 - 100 mg/ml of the antigen binding protein; 20 - 40 mM of histidine; 50- 100 mM of arginine; 0.002 - 0.02% Polysorbate 80 (w/v); 50- 150 mM NaCl; and 0.1 - 2.5% sucrose w/v; wherein pH of the formulation is 6.5 ± 0.5; and wherein the resulting formulation is stable at 2-8 deg C for at least 9 months, at 25 deg C for at least 1 month, at 40 deg C for at least 40 days, at 50 deg C for at least 2 days.

A second aspect of the present disclosure relates to a pharmaceutical formulation comprising: 1 - 100 mg/ml of at least one antigen binding protein, wherein the antigen binding protein is an anti-Dengue monoclonal antibody according to SEQ ID 1 and SEQ ID 2 or wherein the antigen binding protein is an anti-Rabies monoclonal antibody according to SEQ ID 3 and SEQ ID 4; 20 - 40 mM of histidine; 50 - 100 mM of arginine; 0.002 - 0.02% Polysorbate 80 (w/v); 50-150 mM NaCl; 0.1 - 2.5% sucrose w/v; wherein pH of the formulation is 6.5 ± 0.5, wherein the osmolality of the formulation is 300 - 450 mOsmol/kg and viscosity less than 2.5 mPa-S and said formulation is stable at 2-8 deg C for at least 9 months, at 25 deg C for at least 1 month, at 40 deg C for at least 40 days, at 50 deg C for at least 2 days.

A third aspect of the present disclosure relates to a pharmaceutical formulation according to the present disclosure for use in the treatment, prevention or diagnosis of dengue or rabies virus.

Other objectives and features of the present disclosure will appear from the following description, from the drawings, as well as from the attached claims.

### List of Figures:

1. Figure 1: Flow chart - Downstream processing for purification of monoclonal antibody
2. Figure 2: Flow chart - Formulation process for monoclonal antibody

### Description

A first aspect of the present disclosure relates to a method of manufacturing pharmaceutical antigen binding protein with high yield and minimum aggregation, wherein the antigen binding protein is an anti-Dengue monoclonal antibody according to SEQ ID 1 and SEQ ID 2 or wherein the antigen binding protein is an anti-Rabies monoclonal antibody according to SEQ ID 3 and SEQ ID 4, the method comprising the steps of: a) culturing in large scale mammalian cells that express antigen binding protein in a cell culture production medium, wherein the culturing step effectively maintains the cell count in the range of 10 x 10⁶ - 20 x 10⁶ cells/ml and results in a yield of at least 2 gm/L; wherein the cell line of mammalian cells is CHO-K1 SV GS-KO when the antigen binding protein is an anti-Dengue monoclonal antibody and the cell line of mammalian cells is GS-CHO when the antigen binding protein is an anti-Rabies monoclonal antibody; wherein the culturing step includes use of basal medium, use of concentrated basal medium as feed solution, use of feed solutions along-with a definite feeding strategy, resulting in enhanced cell growth, maintaining lower concentrations of lactate and ammonia, and effectively maintaining the cell count thereby increasing cell longevity and high yield; b) purification of antigen binding protein from harvested supernatant obtained in step (a), wherein the purification results in recovery of at least 80% and purity of at least 99%; and wherein the purification comprises affinity chromatography, Low pH viral inactivation, cation exchange chromatography, anion exchange chromatography, nanofiltration, Tangential flow filtration/Ultrafiltration; in a sequential manner; wherein the affinity chromatography matrix is Protein A; wherein the salt concentration of the buffers used in purification is in the range of 30 mM - 500 mM; and c) preparing a stable formulation comprising the antigen binding protein, wherein osmolality of the formulation is in range of 300 - 400 mOsm/Kg and the viscosity of the formulation is less than 2.5 mPa-S, wherein the formulation comprises at least one antigen binding protein, at least one stabilizer, at least one buffering agent, at least one tonicity agent, and at least one surfactant; wherein the formulation comprises 1 - 100 mg/ml of the antigen binding protein; 20 - 40 mM of histidine; 50 - 100 mM of arginine; 0.002 - 0.02% Polysorbate 80 (w/v); 50- 150 mM NaCl; and 0.1 - 2.5% sucrose w/v; wherein pH of the formulation is 6.5 ± 0.5; and wherein the resulting formulation is stable at 2-8 deg C for at least 9 months, at 25 deg C for at least 1 month, at 40 deg C for at least 40 days, at 50 deg C for at least 2 days.

The antigen binding protein has binding affinity towards epitopes present on Dengue virus or Rabies virus.

The isoelectric point (pl) of said antigen binding protein may be 7.5 - 8.5, more preferably 7.8 to 8.2, most preferably 8.12.

The cell line used for the expression of the antigen binding proteins is Chinese Hamster Ovary cells; more particularly the mammalian cell line is CHOK1SV GS-KO when the antigen binding protein is an anti-Dengue monoclonal antibody and the cell line of mammalian cells is GS-CHO when the antigen binding protein is an anti-Rabies monoclonal antibody.

According to one embodiment, the cell culture medium is supplemented with one or more other nutrients, at least once during the culturing step.

According to another embodiment, the cell culture production medium is supplemented on a schedule comprising supplementation that is continuous, daily, every other day, every two days or a combination thereof.

According to a further embodiment, the cells are cultivated in a batch, fed batch, continuous mode, perfusion mode; more particularly in a fed batch mode. It is very well understood, that a person skilled in the art can modulate a process herein according to available facilities and individual needs. More particularly, the cell culture process may be carried out in fed batch mode providing enhanced cell growth, cell longevity and increased protein expression i.e. provides a harvest yield of at least 2 gm/L, preferably in the range of 3 gm/L to 6 gm/L.

The cell culture may be conducted in a flask, a bioreactor, a tank bioreactor, a bag bioreactor or a disposable bioreactor. Preferably, said bioreactor is selected from the group of stirred tank bioreactor, a bubble column bioreactor, an air lift bioreactor, a fluidized bed bioreactor or a packed bed bioreactor; and the said bioreactor has a volume selected from 1L, 2L, 3L, 5L, 10L, 20L, 100L, 200L, 250L, 350L, 500 L, 1000L, 1500L, 3000L, 5000L, 10000L , 20000L and 30,000 liters.

The cell culture media and methods may be used to increase antibody yield by 5%, 10%, 15%, 20%, 30%, 40%, 50%, 60%, 70%, 80%, 90%, 100%, 110%, 120%, 130%, 140%, 150%, 160%, 180%, or 200% , most preferably 40% to 60% as measured over a course of a fortnight. The time period of the fed batch method can be 12 to 20 days; 15 to 20 days or 15 to 18 days.

The cell culture medium may be selected from the group comprising one or more of CD CHO, CD OptiCHO^{™}, CD FortiCHO^{™} (Life Technologies); Ex-Cell^{™} CD CHO (Sigma Aldrich); ProCHO^{™}5 (Lonza); BalanCD^{™} CHO Growth A (Irvine Scientific); CDM4Mab(Hyclone); Cellvento^{™} CHO-100 (EMD Millipore); Cell vento 200 (Merck Millipore); Cell vento 220 (Merck Millipore); Actipro (Hyclone); and combination thereof. Preferably, the cell culture medium is selected from Cell Vento 220 (Merck), ACTIPRO (HyClone/GE), or Gibco^{™} Dynamis^{™} Medium (Thermo Fisher).

The cell culture medium may further be supplemented with glucose and other feed solutions so as to increase cell growth, cell longevity, protein expression and yield. It is very well understood in the art that the feed solutions may be supplemented in rapid bolus or gradual drip manner.

According to one example, the supplementation of feed solution in cell culture medium is performed with a feeding strategy comprising of:
- Initial feeding with Feed solution A, at 0.05% to 0.5% reactor volume, preferably at 0.1% to 0.2% reactor volume, from day 4;
- Feeding with Feed solution A, at 0.1% to 0.5% reactor volume on day 6, 8, 10,11 and 13;
- Feed solution B at less than 8% reactor volume, from day 2 to day 14, on alternate day or in continuous manner;
- Feeding with Feed solution C at less than 8% reactor volume for at least 2 consecutive days beginning from day 2 or day 3, having an intermittent gap of 2 consecutive days, till day 12^{th} or day 14^{th} or day 15^{th} or day 16^{th} or day 18^{th}.
- Feeding with Feed solution D at less than 0.5% reactor volume for at least 2 consecutive days beginning from day 4 or day 5, on alternate day or in continuous manner or having an intermittent gap of 2 consecutive days, till day 12^{th} or day 14^{th} or day 15^{th} or day 16^{th} or day 18^{th}. Optionally feeding at least one feed solution selected from EfficientFeed^{™} A, EfficientFeed^{™} B, EfficientFeed^{™} C, and Dow corning Antifoam C.

Preferably, said Feed solution A, Feed solution B, Feed solution C, Feed solution D is selected one or more from group comprising of Glucose, Cell Boost^{™} 5 Supplement (Hyclone), EX-CELL 293 (Sigma Aldrich), Cell Boost 7a and 7b supplements (Hyclone), 3X Actipro (Hyclone/GE), Cell Vento 220 (1X medium), EX-CELL^{®} Advanced^{™} CHO Feed 1, EfficientFeed^{™} A, EfficientFeed^{™} B, and EfficientFeed^{™} C, and combination thereof.

More preferably, said Feed solution A is Cell Boost^{™} 5 Supplement (Hyclone); Feed solution B is EX-CELL 293 (Sigma Aldrich), Feed solution C is Cell Boost 7a supplements (Hyclone), Feed solution D is Cell Boost 7b supplements (Hyclone). Further, the cell culture medium is supplemented with 10% "3X Actipro" (Hyclone) on 3^{rd} day and 8% Cell Vento 220 (1X medium) on 7^{th} day of cell culturing. It is very well understood that all the feedings addition can vary by ± 1% and ± 1 day by a person skilled in the art.

Cell culture conditions may be employed for increasing the cell growth and longevity and protein expression. The following cell culture conditions employed during the process includes but not limited to:
- pH of cell culture medium is in the range of 6.5 and 7.5;
- Osmolality of the culture medium is in the range of 250 - 500 mOsm/kg; more preferably 400 - 500 mOsm/kg.
- Dissolved oxygen is in the range of 10 - 60%; preferably 20-40%; more preferably 30%.
- Cell culture temperature is in the range of 30°C to 38°C; first temperature preferably 36 - 37 °C and optionally second temperature preferably 30 - 35 °C.
- Glucose concentration is maintained below 7%; preferably between 4% and 5%.
- Harvesting the cell culture when viability is decreased to 80 %;
wherein the cell culture conditions are maintained in a manner that secondary metabolites such as Lactate concentration is not more than 5g/L; and Ammonia concentration is not more than 5 mMol/L.

According to one embodiment, the cell culture medium has an osmolality in range of 250 - 500mOsm/Kg; pH in range of 6.5 - 7.5; dissolved oxygen is maintained in range of 10 - 60%; cell culture temperature is in the range of 30°C to 38°C; first temperature preferably 36 - 37 °C and optionally second temperature preferably 30 - 35 °C; glucose concentration is maintained below 7%; preferably between 4% and 5% ; harvesting the cell culture when viability is decreased to 80 %; wherein the cell culture conditions are maintained in a manner that secondary metabolites such as lactate concentration is not more than 5g/L; and ammonia concentration is not more than 5 mMol/L. Preferably, the osmolality of the fermentation medium is 400 - 500 mOsm/kg.

In another embodiment, the dissolved oxygen of the fermentation medium is maintained in the range of 20 - 40%.

The antigen binding protein obtained from the cell culture harvest is subjected to a purification process comprising affinity chromatography, low pH Viral inactivation, cation exchange chromatography, anion exchange chromatography, nanofiltration, tangential flow filtration/ultrafiltration; in a sequential manner; wherein the affinity chromatography matrix is Protein A; wherein the salt concentration of the buffers used in purification is in the range of 30 mM - 500 mM.

According to a specific embodiment, the salt concentration of the buffers used in purification is in the range of 50 mM - 300 mM.

The purification results in recovery of at least 80% and purity of at least 99%. Impurities including residual cell DNA, residual cell protein, and residual protein A in final purified therapeutic protein is less than 1%.

Problems of aggregation of antigen binding protein during downstream processing of the protein may be mitigated by using i) Salt in an affinity chromatography wash step and ii) Linear gradient of salt solution for elution in ion exchange chromatography step. Preferably, the salt concentration of the buffers used in purification is in the range of 30 mM - 500 mM, more preferably the salt concentration of the buffers used in purification is in the range of 50 mM - 300 mM.

The affinity chromatography used is Protein A chromatography.

The resin used for Protein A chromatography may be selected from the group comprising one or more of Eshmuno A, KanCapATM, MabSelect SuRe^{™}, MabSelect SuRe LX, MabSelect Xtra, rProtein A Sepharose Fast Flow, Poros^{®} MabCapture A, Amsphere^{™} Protein A JWT203, ProSep HC, ProSep Ultra, and ProSep Ultra Plus. Preferably the Protein A affinity chromatography resin is MabSelect SuRe^{™}, Eshmuno A, Kancap A or Poros MabCapture. More preferably the Protein A affinity chromatography resin is MabSelect SuRe^{™}.

The wash buffer used for Protein A chromatography may be selected from the group comprising one or more of
- 10 - 30 mM Phosphate buffer, preferably 20 mM Phosphate buffer; 100 - 150 mM NaCl, preferably 150 mM NaCl; 0.05% Polysorbate 80; pH 7.0 ± 0.2;
- 10 - 30 mM Phosphate buffer, preferably 20 mM Phosphate buffer; 250 mM - 1 M NaCl, preferably 1M NaCl; 0.05% Polysorbate 80; pH 7.0 ± 0.2;
- 1-30 mM Phosphate buffer, preferably 10 mM Phosphate buffer; 100- 150 mM NaCl, preferably 125mM NaCl; 0.05% Polysorbate 80; pH 7.0 ± 0.2.

The elution buffer used for Protein A chromatography may comprise of 10 - 30 mM Citrate buffer; pH 3.0 ± 0.5; and optionally 0.01 - 0.05 % (w/v) Polysorbate 80; preferably the elution buffer comprises of 20mM Citrate buffer; pH 3.0 ± 0.2; and optionally 0.025 % (w/v) Polysorbate 80.

According to one embodiment, the protein A chromatography comprises: a) Equilibration buffer: 20 mM phosphate Buffer; 100 - 150 mM NaCl; 0.05% Polysorbate 80; pH 7.0 ± 0.2; b) Loading : Clarified Harvest; c) Wash I Buffer: 20 mM phosphate buffer; 100 - 150 mM NaCl, more particularly 150mM; 0.05% Polysorbate 80; pH 7.0 ± 0.2; d) Wash II Buffer: 20 mM phosphate buffer; 250 mM - 1 M NaCl, more particularly 1M; 0.05% Polysorbate 80; pH 7.0 ± 0.2; e) Wash III Buffer: 10 mM phosphate buffer; 100 - 150 mM NaCl, more particularly 125mM; 0.05% Polysorbate 80; pH 7.0 ± 0.2; f) Elution Buffer: 20mM citrate buffer; pH 3.0 ± 0.2; and optionally 0.025 % (w/v) Polysorbate 80; g) CIP Buffer: 0.1M NaOH; h) residence time: 4.00 - 8.00 minutes; i) column used: XK26; j) linear flow rate is 10 - 500 cm/hr, more particularly 100-150 cm/hr.

Eluate obtained from the affinity chromatography step is subjected to low pH viral inactivation. It is very well understood in the art that viral inactivation and reduction of the eluate may be effected by method selected individually or in combination from the group comprising of pH treatment, detergent treatment, heat treatment, and virus reduction filtration.

According to one embodiment, viral inactivation of the eluate from the protein A affinity chromatography is accomplished by holding the eluate at pH 3.3 - 3.5 for 50 - 100 minutes.

The eluate may be pH neutralized by subjecting it to neutralization buffer i.e. 1 M Tris/Citrate buffer pH 7.0 ± 0.2. It is very well understood in the art that any other compatible buffer may be used alternatively for effective pH neutralization of the eluate.

The viral inactivated eluate is subjected to cation exchange chromatography and anion exchange. This chromatography may be carried out in "bind and elute" mode or "flow through" mode. Preferably, cation exchange chromatography and anion exchange chromatography is carried out in any sequential order. The chromatography resin may be a multi-modal resin like Capto MMC resin (GE Healthcare).

According to one embodiment, the cation exchange chromatography is conducted using a resin selected from the group comprising one or more of sulfonate based group; a sulfoethyl based group; a sulphopropyl based group; a sulfoisobutyl based group; a sulfoxy ethyl based group, a carboxymethyl based group; sulfonic and carboxylic acid based groups; a carboxylic acid based group; a sulfonic acid based group; and an orthophosphate based group.

Preferably, the cation chromatography parameters including chromatography resin and buffer conditions are selected in such a manner that the positively charged protein binds to the chromatography resin while the negatively charged molecules comes in the flow through, further proteins are subjected to elution using a salt gradient.

Preferably, the cation exchange chromatography resin is selected from the group comprising one or more of sulfonate based group (e.g., MonoS, MiniS, Source 15S and 30S, SP SEPHAROSE^{®} Fast Flow, SP SEPHAROSE^{®} High Performance from GE Healthcare, TOYOPEARL^{®} SP-650S and SP-650M from Tosoh, MACRO-PREP^{®} High S from BioRad, Ceramic HyperD S, TRISACRYL^{®} M and LS SP and Spherodex LS SP from Pall Technologies); a sulfoethyl based group (e.g., FRACTOGEL^{®} SE, from EMD, POROS^{®} S-10 and S-20 from Applied Biosystems); a sulphopropyl based group (e.g., TSK Gel SP 5PW and SP-5PW- HR from Tosoh, POROS^{®} HS-20, HS 50, and POROS^{®} XS from Life Technologies); a sulfoisobutyl based group (e.g., FRACTOGEL^{®} EMD S03 " from EMD); a sulfoxy ethyl based group (e.g., SE52, SE53 and Express-Ion S from Whatman), a carboxymethyl based group (e.g., CM SEPHAROSE^{®} Fast Flow from GE Healthcare, Hydrocell CM from Biochrom Labs Inc., MACRO-PREP^{®} CM from BioRad, Ceramic HyperD CM, TRISACRYL^{®} M CM, TRISACRYL^{®} LS CM, from Pall Technologies, Matrex CELLUFINE^{®} C500 and C200 from Millipore, CM52, CM32, CM23 and Express-Ion C from Whatman, TOYOPEARL^{®} CM-650S, CM-650M and CM-650C from Tosoh); sulfonic and carboxylic acid based groups (e.g., BAKERBOND^{®} Carboxy-Sulfon from J.T. Baker); a carboxylic acid based group (e.g., WP CBX from J.T Baker, DOWEX^{®} MAC-3 from Dow Liquid Separations, AMBERLITE^{®} Weak Cation Exchangers, DOWEX^{®} Weak Cation Exchanger, and DIAION^{®} Weak Cation Exchangers from Sigma- Aldrich and FRACTOGEL^{®} EMD COO- from EMD); a sulfonic acid based group (e.g., Hydrocell SP from Biochrom Labs Inc., DOWEX^{®} Fine Mesh Strong Acid Cation Resin from Dow Liquid Separations, UNOsphere S, WP Sulfonic from J.T. Baker, SARTOBIND^{®} S membrane from Sartorius, AMBERLITE^{®} Strong Cation Exchangers, DOWEX^{®} Strong Cation and DIAION^{®} Strong Cation Exchanger from Sigma-Aldrich); and an orthophosphate based group (e.g., PI 1 from Whatman).

More preferably, the resin used for cation exchange chromatography is Fractogel^{®} EMD SO₃⁻, Fractogel^{®} EMD SE Hicap (Merck), CMM HyperCel^{™} (Pall Corporation), Capto S ImpAct.

The process parameters for cation exchange chromatography include but not limited to Pre-equilibration buffer [200 mM Citrate buffer; pH 6.0 ± 0.2]; Equilibration buffer [10 mM Citrate buffer; Polysorbate 80 (0.025 % (w/v)); pH 6.0 ± 0.2]; Low pH hold for neutralization; Wash Buffer A [10 mM Citrate buffer; pH 6.0 ± 0.2]; Wash buffer B [20 mM Citrate buffer; 300 - 500 mM NaCl; pH 6.0 ± 0.2]; CIP buffer [0.5M NaOH]; Residence time [4.00 - 7.00 minutes]; Column used [XK26].

Thus, according to one embodiment, the cation exchange chromatography comprises a) Pre-equilibration buffer: 200 mM citrate buffer; pH 6.0 ± 0.2; b) Equilibration buffer: 10 mM citrate buffer; 0.025 % (w/v) Polysorbate 80; pH 6.0 ± 0.2; c) Wash Buffer A: 10 mM citrate buffer; pH 6.0 ± 0.2; d) Wash buffer B: 20 mM citrate buffer; 300 - 500 mM NaCl; pH 6.0 ± 0.2; e) CIP buffer: 0.5M NaOH; f) Residence time: 4.00 - 7.00 minutes; g) Column used: XK26.

The viral inactivated eluate is also subjected to anion exchange chromatography. Preferably, the anion chromatography parameters including chromatography resin and buffer conditions are selected in such a manner that all negatively charged impurities are bound with the membrane while the therapeutic protein elutes in a flow through.

Preferably, the anion exchange chromatography resin is selected from the group comprising one or more of DEAE cellulose, POROS^{®} PI 20, PI 50, HQ 10, HQ 20, HQ 50, D 50 from Applied Biosystems, SARTOBIND^{®} Q from Sartorius, MonoQ, MiniQ, Source 15Q and 30Q, Q, DEAE and ANX SEPHAROSE^{®} Fast Flow, Q SEPHAROSE, Q SEPHAROSE^{®} High Performance, QAE SEPHADEX^{®} and FAST Q SEPHAROSE^{®} (GE Healthcare),WP PEI, WP DEAM, WP QUAT from J.T. Baker, Hydrocell DEAE and Hydrocell QA from Biochrom Labs Inc., U Osphere Q, MACRO-PREP^{®} DEAE and MACRO-PREP^{®} High Q from Biorad, Ceramic HyperD Q, ceramic HyperD DEAE, TRISACRYL^{®} M and LS DEAE, Spherodex LS DEAE, QMA SPHEROSIL^{®} LS, QMA SPHEROSIL^{®} M and MUSTANG^{®} Q from Pall Technologies, DOWEX^{®} Fine Mesh Strong Base Type I and Type II Anion Resins and DOWEX^{®} MONOSPHER E 77, weak base anion from Dow Liquid Separations, INTERCEPT^{®} Q membrane, Matrex CELLUFINE^{®} A200, A500, Q500, and Q800, from Millipore, FRACTOGEL^{®} EMD TMAE, FRACTOGEL^{®} EMD DEAE and FRACTOGEL^{®} EMD DMAE from EMD, AMBERLITE^{®} weak strong anion exchangers type I and II, DOWEX^{®} weak and strong anion exchangers type I and II, DIAION^{®} weak and strong anion exchangers type I and II, DUOLITE^{®} from Sigma-Aldrich, TSK gel Q and DEAE 5PW and 5PW-HR, TOYOPEARL^{®} SuperQ-650S, 650M and 650C, QAE-550C and 650S, DEAE-650M and 650C from Tosoh, QA52, DE23, DE32, DE51, DE52, DE53, Express-Ion D and Express- Ion Q from Whatman; more preferably Anion-exchange chromatography resin is selected from Sartobind Q (Sartorius), Eshmuno Q (Merck), MUSTANG^{®} Q (Pall Corporation) and Poros X (Thermo).

The process parameters for anion exchange chromatography includes but not limited to Cleaning buffer [0.5M NaOH]; Pre-equilibration buffer [200 mM Citrate buffer; pH 6.0 ± 0.2]; Equilibration buffer [20 mM Citrate buffer; pH 6.0 ± 0.2; and optionally 0.025% Polysorbate 80]; Storage buffer [0.1M NaOH]; Linear Flow rate [10 - 500 cm/hr, more particularly 100-150 cm/hr]; Column used [XK26].

Thus, according to one embodiment, the anion exchange chromatography comprises a) Cleaning buffer: 0.5M NaOH; b) Pre-equilibration buffer: 200 mM citrate buffer; pH 6.0 ± 0.2; c) Equilibration buffer: 20 mM citrate buffer; pH 6.0 ± 0.2; and optionally 0.025% Polysorbate 80; d) Storage buffer: 0.1M NaOH; e) Linear Flow rate is 10 - 500 cm/hr, more particularly 100-150 cm/hr; f) Column used: XK26.

According to another embodiment, the anion exchange chromatography is "flow through and wash mode" or "bind and elute mode".

According to one embodiment, the method according to the present disclosure may further comprise an additional chromatography step selected from the group comprising one or more of Hydrophobic interaction chromatography, Hydrophobic charge induction chromatography, Ceramic hydroxyapatite chromatography, Multimodal chromatography (Capto MMC and Capto Adhere), Membrane chromatography (Q membranes including Intercept^{™} (Millipore), Mustang^{®} (Pall Corporation) and Sartobind^{™} (Sartorius)).

In the method according to the present disclosure, virus particles can be removed by using 20 nm filter.

According to one embodiment, the removal of viral particles is accomplished by nanofiltration using virus retentive filter. The virus retentive filter may be selected from the group of Viresolve PRO (Merck), Planova 20N (Asahi Kasei), Bio EXL PALL PEGASUS PRIME, PEGASUS SV4 (Pall Life Sciences), and Virosart (Sartorius), Virosart CPV filter from Sartorius, Virosolve from Millipore, Ultipor DV20 or DV50 from Pall, Planova 20N and 50N or BioEx from Asahi. It is very well understood in the art that any other filter having retention capacity for viruses may be used in this step. Preferably the filter used for removal of viral particles is selected from Viresolve PRO (Merck), Bio EXL PALL PEGASUS PRIME, PEGASUS SV4 (Pall Life Sciences), and Virosart (Sartorius).

The therapeutic protein is concentrated to a desired concentration and buffer exchanged in formulation buffer. The buffer is exchanged in a tangential flow filtration system or an ultra flow filtration system.

According to one embodiment, the antigen binding protein is concentrated using Tangential Flow Filtration (TFF).

According to another embodiment, the TFF is carried out using 30 kDa membrane.

The parameters of Tangential flow filtration comprise of one or more selected from Diafiltration using diafiltration buffer [25 mM Histidine buffer; 75 mM Arginine buffer; 50 - 150 mM NaCl; pH 6.50 ± 0.5]; Cleaning buffer [0.5M NaOH]; Storage buffer [0.1 M NaOH]; Equilibration using 5 - 10 X membrane volume; Concentration and Diafilteration using 10-20 diafilteration volume; WFI wash using 3-5 membrane volume; cleaning using 0.5 - 1.0 M NaOH; Storage [0.1M NaOH].Preferably, Tangential flow filtration is carried out using 30 kDa MWCO membrane selected from the group comprising one or more of Centramate T series PES membrane (Pall Corporation), Hydrosart (Sartorius), and Pelicon 3 (Merck).

According to a specific embodiment, the Tangential Flow Filtration process comprises a) Diafiltration using diafiltration buffer: 25 mM histidine buffer; 75 mM arginine buffer; 50 -150 mM NaCl; pH 6.50 ± 0.5; b) Cleaning buffer: 0.5M NaOH; c) Storage buffer: 0.1M NaOH; d) Equilibration using 5 -10 X membrane volume; e) Concentration and diafiltration using 10 - 20 diafiltration volume; f) WFI wash using 3-5 membrane volume; g) Cleaning using 0.5 - 1.0 M. The method of the present disclosure comprises the step of c) preparing a stable formulation comprising the antigen binding protein, wherein osmolality of the formulation is in range of 300 - 400 mOsm/Kg and the viscosity of the formulation is less than 2.5 mPa-S, wherein the formulation comprises at least one antigen binding protein, at least one stabilizer, at least one buffering agent, at least one tonicity agent, and at least one surfactant. The formulation comprises 1 - 100 mg/ml of the antigen binding protein; 20 - 40 mM of histidine; 50-100 mM of arginine; 0.002 - 0.02% Polysorbate 80 (w/v); 50 - 150 mM NaCl; and 0.1 - 2.5% sucrose w/v; wherein pH of the formulation is 6.5 ± 0.5; and wherein the resulting formulation is stable at 2-8 deg C for at least 9 months, at 25 deg C for at least 1 month, at 40 deg C for at least 40 days, at 50 deg C for at least 2 days.

According to one embodiment of the method of the present disclosure, the purified therapeutic protein preparation contains no greater than 2% aggregates, preferably less than 1% aggregates.

According to another embodiment of the method of the present disclosure, the stable antigen binding protein formulation comprises 1 mg/ml to 100 mg/ml of antigen binding protein.

According to yet another embodiment of the method of the present disclosure, the antigen binding protein formulation comprises of not more than 3% aggregation, minimum amount of sub-visible particles and improved potency.

According to a further embodiment of the method of the present disclosure, the concentration of the antigen binding protein monomer in the formulation is greater than 99%; residual CHO DNA is not more than 2 pg/mg of antigen binding protein, more particularly not more than 0.1 pg/mg of antigen binding protein; residual CHO protein is not more than 100 ng/mg of antigen binding protein, more particularly not more than 10 ng/mg of antigen binding protein; residual Protein-A is not more than 10 ng/mg of antigen binding protein, more particularly not more than 1.5 ng/mg of antigen binding protein; Endotoxin is not more than 0.1 EU/mg of antigen binding protein.

According to yet another embodiment of the method of the present disclosure, formulation comprises <1% (w/v), most preferably 0.5% (w/v), sucrose; 25 mM, histidine; 75 mM, arginine; 100 - 145 mM, sodium chloride; 0.02%( w/v), Polysorbate 80; and 1 mg/ml to 50 mg/ml, antigen binding protein.

In a second aspect, the present disclosure relates to a pharmaceutical formulation comprising a) 1 - 100 mg/ml of at least one antigen binding protein, wherein the antigen binding protein is an anti-Dengue monoclonal antibody according to SEQ ID 1 and SEQ ID 2 or wherein the antigen binding protein is an anti-Rabies monoclonal antibody according to SEQ ID 3 and SEQ ID 4; b) 20 - 40 mM of histidine; c) 50 - 100 mM of arginine; d) 0.002 - 0.02% Polysorbate 80 (w/v); e) 50 - 150 mM NaCl; f) 0.1 - 2.5% sucrose w/v; wherein pH of the formulation is 6.5 ± 0.5, wherein the osmolality of the formulation is 300 - 450 mOsmol/kg and viscosity less than 2.5 mPa-S and said formulation is stable at 2-8 deg C for at least 9 months, at 25 deg C for at least 1 month , at 40 deg C for at least 40 days, at 50 deg C for at least 2 days.

According to one embodiment, the pharmaceutical formulation comprises 2 - 80 mg/ml of the least one antigen binding protein; 25 mM of histidine; 75 mM of arginine; 101 mM NaCl; 0.02% Polysorbate 80 (w/v); and 0.5% (w/v) sucrose; wherein pH of the formulation is 6.5 ± 0.5.

A the osmolality of the formulation is 380 mOsmol/kg.

According to one embodiment, the pharmaceutical formulation comprises 2 - 80 mg/ml antigen binding protein; 25 mM of histidine; 75 mM of arginine; 101 mM NaCl; 0.02% Polysorbate 80 (w/v); and 0.5% sucrose w/v; wherein pH of the formulation is 6.5 ± 0.5 osmolality 380 mOsm/Kg, viscosity less than 2.5 mPa-S.

According to one embodiment, the pharmaceutical formulation comprises 25 mg/ml antigen binding protein; 25 mM of histidine; 75 mM of arginine; 101 mM NaCl; 0.02% Polysorbate 80 (w/v); and 0.5% sucrose w/v; wherein pH of the formulation is 6.5 ± 0.5 osmolality 380 mOsm/Kg, viscosity less than 2.5 mPa-S.

According to one embodiment, the pharmaceutical formulation comprises 50 mg/ml antigen binding protein; 25 mM of histidine; 75 mM of arginine; 101 mM NaCl; 0.02% Polysorbate 80 (w/v); and 0.5% sucrose w/v; wherein pH of the formulation is 6.5 ± 0.5 osmolality 380 mOsm/Kg, viscosity less than 2.5 mPa-S.

According to another embodiment, the formulation is a liquid formulation.

The purified antigen binding protein may be formulated with pharmaceutical excipients.

Thus, the antigen binding protein formulation comprises at least one antigen binding protein, at least one stabilizer (sucrose), at least one buffering agent (histidine and arginine), at least one tonicity agent (sodium chloride, NaCl), and at least one surfactant (Polysorbate 80). Optionally, formulation comprises of a preservative.

The formulation may comprise a further stabilizer selected from the group comprising of one or more of sorbitol, trehalose, mannitol, dextran, inositol, glucose, fructose, lactose, xylose, mannose, maltose, Raffinose and combination thereof. Preferably, the formulation comprises <1% sucrose w/v.

The formulation may comprise a further buffering agent selected from the group comprising of one or more of glycine, sodium citrate, sodium phosphate, citric acid, HEPES, potassium acetate, potassium citrate, potassium phosphate, sodium acetate, sodium bicarbonate, Tris base, or Tris-HCI, and combination thereof. The buffering agents provide a pH of 6.0 to 7.0. The buffering agents provide a pH of 6.0 to 7.0, preferably 6.3 to 6.8, or 6.5.

Preferably, the formulation comprises Histidine at a concentration of 25 mM.

Preferably, the formulation comprises Arginine at a concentration of 70 to 80 mM.

The formulation may comprise a further tonicity agent selected from the group comprising of one or more of dextrose, glycerin, mannitol, and potassium chloride. Preferably, the formulation comprises NaCl at a concentration of 100 - 145 mM.

The formulation may comprise a further surfactant selected from the group comprising of one or more of polysorbates (e.g. polysorbate- 20); poloxamers (e.g. poloxamer 188); Triton; sodium dodecyl sulfate (SDS); sodium laurel sulfate; sodium octyl glycoside; lauryl-, myristyl-, linoleyl-, or stearyl-sulfobetaine; lauryl-, myristyl-, linoleyl-or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (e.g. lauroamidopropyl); myristamidopropyl-, palmidopropyl-, or isostearamidopropyl-dimethylamine; sodium methyl cocoyl-, or disodium methyl oleyl-taurate; and the MONAQUAT^{®} series (Mona Industries, Inc., Paterson, N.J.), polyethyl glycol, polypropyl glycol, and copolymers of ethylene and propylene glycol (e.g. Pluronics, PF68 etc). Preferably, the formulation comprises Polysorbate 80 at a concentration of 0.02% (w/v).

Preferably, the formulation comprises the antigen binding protein at a concentration of 1mg/L to 50 mg/L, 20 mg/L to 40 mg/L. Most preferably the formulation comprises the antigen binding protein at a concentration of 1mg/L to 50 mg/L.

The formulation may further comprise a preservative. The preservative may be selected from the group comprising of benzyl alcohol, m-cresol, and phenol.

According to one example, the pharmaceutical formulation comprises of 2 - 80 mg/ml of anti-Dengue monoclonal antibody; 25 mM of Histidine; 75 mM of Arginine; 101 mM NaCl; 0.02% Polysorbate 80 (w/v); and 0.5% Sucrose w/v; wherein pH of the formulation is 6.5 ± 0.5 Osmolality 380 mOsm/Kg, viscosity less than 2.5 mPa-S.

According to another example, the pharmaceutical formulation comprises of 25 mg/ml of anti-Dengue monoclonal antibody; 25 mM of Histidine; 75 mM of Arginine; 101 mM NaCl; 0.02% Polysorbate 80 (w/v); and 0.5% Sucrose w/v; wherein pH of the formulation is 6.5 ± 0.5 ,Osmolality 380 mOsm/Kg, viscosity less than 2.5 mPa-S.

According to yet another example, the pharmaceutical formulation comprises of 50 mg/ml of anti-Dengue monoclonal antibody; 25 mM of Histidine; 75 mM of Arginine; 101 mM NaCl; 0.02% Polysorbate 80 (w/v); and 0.5% Sucrose; wherein pH of the formulation is 6.5 ± 0.5 Osmolality 380 mOsm/Kg, viscosity less than 2.5 mPa-S.

According to a further example, the pharmaceutical formulation comprises of 2 - 80 mg/ml of anti-Rabies monoclonal antibody; 25 mM of Histidine; 75 mM of Arginine; 101 mM NaCl; 0.02% Polysorbate 80 (w/v); and 0.5% Sucrose w/v; wherein pH of the formulation is 6.5 ± 0.5 Osmolality 380 mOsm/Kg, viscosity less than 2.5 mPa-S.

According to yet a further example, the pharmaceutical formulation comprises of 25 mg/ml of anti-Rabies monoclonal antibody; 25 mM of Histidine; 75 mM of Arginine; 101 mM NaCl; 0.02% Polysorbate 80 (w/v); and 0.5% Sucrose w/v; wherein pH of the formulation is 6.5 ± 0.5 Osmolality 380 mOsm/Kg, viscosity less than 2.5 mPa-S.

According to one example, the pharmaceutical formulation comprises of 50 mg/ml of Rabies monoclonal antibody; 25 mM of Histidine; 75 mM of Arginine; 101 mM NaCl; 0.02% Polysorbate 80 (w/v); and 0.5% Sucrose w/v; wherein pH of the formulation is 6.5 ± 0.5 Osmolality 380 mOsm/Kg, viscosity less than 2.5 mPa-S.

The pharmaceutical formulation may be a lyophilized formulation.

The affinity and potency of the antigen binding protein may be measured by one or more of ELISA or flow cytometry. Preferably, indirect ELISA based method is used to quantify binding of antigen binding protein to the specific antigen. Preferably, Dengue Mab formulation is tested against all serotypes of the dengue viruses and amount of Dengue mAb is determined. The potency of the antigen binding protein is reported as % activity relative to the reference standard. It is very well understood that any other similar method may be used to demonstrate the potency and affinity of the therapeutic protein.

Focus reduction neutralization test (PRNT / FRNT) or a related test may be carried out for evaluating neutralization of viral activity by antigen binding protein. Preferably, Dengue mAb formulation is tested against all serotypes of the dengue viruses and EC50 values are calculated for neutralization of Dengue Viruses. It is very well understood that any other similar method may be used to demonstrate the neutralization activity of the therapeutic protein. HPLC based size exclusion chromatography may be used to assess the presence of aggregates in therapeutic protein formulation. Preferably, Phenomenex Bio-Sec-S 3000 column is used to demonstrate the aggregate and monomer percentage of Dengue mab formulation. It is very well understood that any other similar method may be used to assess the presence of aggregates in therapeutic protein formulation.

The formulation may be stored in a suitable container. The container may be selected from a bottle, a vial, a IV bag, a wearable injector, a bolus injector, a syringe, a pen, a pump, a multidose needle syringe, a multidose pen, a injector, a syrette, an autoinjector, a pre-filled syringe, or a combination thereof.

At least one primary packaging component may comprise a container closure selected from polypropylene (PP), polyethylene terephthalate (PETG), high-density polyethylene (HDPE), polyethylene terephthalate (PET), polypentafluorostyrene (PFS), polycarbonate, polyvinyl chloride (PVC), polyolefin, polycyclopentane (CZ.RTM.), cyclic olefin copolymer (COC), and combinations or copolymers thereof.

The anti-dengue antibody or anti-rabies antibody formulations disclosed herein can be used (alone or in combination with other agents or therapeutic modalities) to treat, prevent and or diagnose dengue or rabies virus. For example, the combination therapy can include an anti-dengue antibody molecule co-formulated with, and/or co-administered with, one or more additional therapeutic agents, e.g., antiviral agents (including other anti-dengue antibodies), vaccines (including dengue virus vaccines), or agents that enhance an immune response. In other embodiments, the antibody molecules are administered in combination with other therapeutic treatment modalities, such as intravenous hydration, fever-reducing agents (such as acetaminophen), or blood transfusion. Such combination therapies may advantageously utilize lower dosages of the administered therapeutic agents, thus avoiding possible toxicities or complications associated with the various monotherapies.

In a third aspect, the present disclosure relates to a pharmaceutical formulation according to the present disclosure for use in the treatment, prevention or diagnosis of dengue or rabies virus.

According to one embodiment, the formulation is comprised in a container selected from a bottle, a vial, an ampule, an IV bag, a wearable injector, a bolus injector, a syringe, a pen, a pump, a multidose needle syringe, a multidose pen, a injector, a syrette, an autoinjector, a pre-filled syringe, or a combination thereof.

According to another embodiment, at least one primary packaging component comprises a container closure selected from polypropylene (PP), polyethylene terephthalate (PETG), high-density polyethylene (HDPE), polyethylene terephthalate (PET), polypentafluorostyrene (PFS), polycarbonate, polyvinyl chloride (PVC), polycyclopentane (CZ.RTM.), cyclic olefin copolymer (COC), polyolefin, and combinations or copolymers thereof.

### Examples:

### Example 1: Upstream process for cell culturing and expression of therapeutic protein i.e. Dengue (VIS513) Monoclonal antibody

### Protocol:

Cell culturing at 10 L scale was carried out in a Fed batch manner using below mentioned parameters during fermentation/upstream process.

The Dengue monoclonal antibody was expressed in cell line "CHO - K1 SV GS-KO" obtained from Visterra Inc. USA.
- Cell culture medium used for cell growth and expression of therapeutic protein i.e. Dengue monoclonal antibody was 1X Cellvento^{™} CHO-220 Liquid Medium.
- Feed solution A, Feed solution B, Feed solution C, Feed solution D selected from group comprising of Glucose, Cell Boost^{™} 5 Supplement (Hyclone), EX-CELL 293 (Sigma Aldrich), Cell Boost 7a and 7b supplements (Hyclone), 3X Actipro (Hyclone), Cell Vento 220 (3X medium), EX-CELL^{®} Advanced^{™} CHO Feed 1 was used for supplementation feed.
- pH of the fermentation medium was maintained at 6.7 to 7.5.
- Osmolality of the fermentation medium was maintained at <490 mOsm/Kg.
- Dissolved oxygen of the fermentation medium was maintained at about 20% to about 40%.
- Temperature of the fermentation medium was maintained at 36.5 ± 0.5.
- The culture was harvested upon drop in cell count to 60%

Feed supplementation was done in a gradual drip manner as per following Table 1:

**Table 1**

| Feeding Strategy protocol Day | Feed A | Feed B | Feed C | Feed D | Basal Medium 1 (3X Actipro" (Hyclone) | Basal Medium 2 (Cellvento^{™} CHO-220 (3X)) |
|---|---|---|---|---|---|---|
| 0 | | | | | | |
| 1 | | | | | | |
| 2 | | 4% | | | | |
| 3 | | | | | 10% | |
| 4 | 0.2% | | 4% | 0.4% | | |
| 5 | | 6% | 4% | 0.4% | | |
| 6 | 0.2% | 4% | 4% | 0.4% | | |
| 7 | | 6% | | | | 8% |
| 8 | 0.2% | | 4% | 0.4% | | |
| 9 | | 4% | 4% | 4.0% | | |
| 10 | 0.1% | 2% | 4% | 0.4% | | |
| 11 | 0.2% | 2% | 2% | 0.2% | | |
| 12 | | 4% | | | | |
| 13 | 0.1% | 2% | 2% | 0.2% | | |
| 14 | | 3% | | | | |
| 15 | | 3% | | | | |
| 16 | | | | | | |

### Results & conclusion:

Viable colony count and Yield obtained during the fermentation process was as follows:

**Table 2 : Viable colony count and Yield obtained during the fermentation process**

| Day | Batch 1 | | | Batch 2 | |
|---|---|---|---|---|---|
| | Viable colony count | Titer gm/L | | Viable colony count | Titer gm/L |
| 0 | 0.8 | | | 0.81 | |
| 1 | 1.5 | | | 1.7 | |
| 2 | 2.8 | | | 3.45 | |
| 3 | 4.3 | | | 5.9 | |
| 4 | 6.5 | | | 7.4 | |
| 5 | 8.4 | | | 11.8 | |
| 6 | 10.1 | 0.54 | | 15.5 | 0.63 |
| 7 | 12.7 | 0.77 | | 18.5 | 0.91 |
| 8 | 14.5 | 1.18 | | 19 | 1.29 |
| 9 | 17 | 1.62 | | 18.5 | 1.76 |
| 10 | 17.25 | 2.15 | | 18.8 | 2.42 |
| 11 | 17.25 | 2.64 | | 18 | 2.85 |
| 12 | 16.8 | 3.05 | | 18 | 3.39 |
| 13 | 15.3 | 3.6 | | 17.2 | 4.0 |
| 14 | 15 | 3.99 | | 17 | 4.32 |
| 15 | 14.7 | 4.43 | | 14.9 | 4.68 |
| 16 | 14.5 | 4.56 | | 13.5 | 4.86 |

Applicant has found that by using Cell culture process comprising of basal medium, concentrated basal medium as feed solution, use of feed solutions along-with a definite feeding strategy, enhanced cell growth, lower concentrations of lactate and ammonia can be obtained thereby effectively maintaining the cell count and increasing cell longevity and high yield.Yield of greater than 4 gm/L was obtained in fermentation process. Harvest obtained was further subjected to purification / downstream processing.

### Example 2:

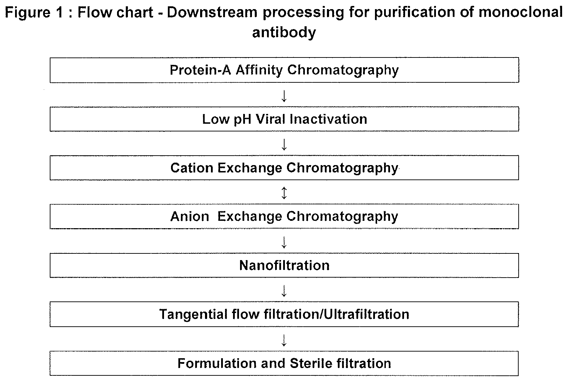

Cell culture obtained in example 1 was harvested and later subjected to protocol for purification of the dengue (VIS513) monoclonal antibody as per Figure 1

Detailed process used was as follows:
Protein-A Affinity Chromatography:
In this step targeted monoclonal antibody was separated from the media components in the harvested supernatant. Clarified supernatant was passed through the chromatography column and then eluted using compatible elution buffers.

Materials used:
**Resin (Matrix):** Mab Select Sure/Eshmuno A (Protein-A Affinity)
**Residence Time:** 4.0-8.0 minutes
**Column used:** XK 26
**Equilibration Buffer:** 20 mM Phosphate Buffer + 150 mM NaCl + 0.05 %(w/v) Polysorbate 80, pH 7.0±0.2.
**Wash I Buffer:** 20 mM Phosphate Buffer + 150mM NaCl + 0.05% (w/v) Polysorbate 80, pH 7.0±0.2.
**Wash II Buffer:** 20 mM Phosphate Buffer + 1M NaCl + 0.05% (w/v) Polysorbate 80, pH 7.0±0.2.
**Wash III Buffer:** 10 mM Phosphate Buffer + 125 mM NaCl + 0.025% (w/v) Polysorbate 80, pH 6.0±0.2.
**Elution Buffer:** 20 mM Citrate buffer + 0.025% (w/v) Polysorbate 80, pH 3.0±0.2.
**CIP Buffer:** 0.1 M NaOH

### Process Parameters used:

**Table 3:**

| S. No. | Process Step | Column Volume | Linear Flow Rate (cm/hr) |
|---|---|---|---|
| 1 | Equilibration | 5 | <300 |
| 2 | Loading (mL) | As Actual | <300 |
| 3 | Wash I | 2 | <300 |
| 4 | Wash II | 4 | <300 |
| 5 | Wash III | 4 | <300 |
| 6 | Elution | 5 | <300 |
| 7 | Cleaning | 3 | <300 |
| 8 | Storage | 2 | <300 |

### 1. Low pH Viral inactivation

i. Eluate from protein-A Affinity chromatography was subjected to low pH i.e. 3.5±0.1 for 60±10 minutes to inactivate the viral particles.
ii. After low pH hold, eluate was neutralized using neutralization buffer i.e. 1 M Tris/Citrate buffer having pH 7.0±0.2.
iii. Conductivity of neutralized eluate was adjusted using WFI with 0.025% (w/v) Polysorbate 80.

### 2. Cation Exchange Chromatography

Positively charged antibody molecules bound with the column while negatively charged molecules come in the flow through. Column bound antibody molecules are eluted using salt gradient.

### Materials used:

**Resin used:** Fractogel SO3⁻/ Fractogel SE Hicap (Merck)
**Residence Time:** 4.00-7.00 minutes
**Column used:** XK 26
**Pre Equilibration:** 200 mM Citrate buffer pH 6.0±0.2.
**Equilibration:** 10 mM Citrate buffer +0.025% (w/v) Polysorbate 80, pH 6.0±0.2.
**Loading:** Low pH Hold neutralized.
**Wash Buffer A:** 10 mM Citrate buffer, pH 6.0±0.2.
**Wash Buffer B:** 20 mM Citrate buffer + 300 mM NaCl, pH 6.0±0.2.
**CIP Buffer:** 0.5 M NaOH
**Storage Buffer:** 0.1 M NaOH
**Process Parameters:**

**Table 4:**

| S. No. | Process Step | CV | Linear Flow Rate (cm/hr) |
|---|---|---|---|
| 1 | Pre Equilibration | 2 | <300 |
| 2 | Equilibration | 5 | <300 |
| 3 | Loading | As Actual | <300 |
| 4 | Wash | 5 | <300 |
| 5 | Elution 0-60% B (Gradient) | 15 | <300 |
| 6 | 100% B | 2 | <300 |
| 7 | CIP | 3 | <300 |

### Fraction collection during gradient

**Table 5:**

| S.No. | Fraction Name | Collection Criteria (UV₂₈₀) mAU |
|---|---|---|
| 1 | Fraction 01 | Upto 300 |
| 2 | Fraction 02 | 300 - 700 |
| 3 | Fraction 03 | 700 till base line |
| 4 | Fraction 04 (100% B) | Baseline to base line |

| | | |
|---|---|---|
| Anion Exchange Chromatography: | | |

All negatively charged impurities are bound with the membrane while antibody comes in the flow through.

Materials used:
**Membrane/Resin used:** Sartobind Q single Sep mini (Sartorius)/Eshmuno Q
Loading volume: 150mg/mL-1000 mg/mL
Column used: XK 26
Cleaning Buffer: 0.5 M NaOH
Pre-equilibration buffer: 200 mM Citrate buffer, pH 6.0 ±0.2.
Equilibration Buffer: 20 mM Citrate buffer pH 6.0±0.2 ; and optionally 0.025% PS-80 pH 6.0±0.2
Storage Buffer: 0.1 M NaOH

### Process Parameters:

**Table 6:**

| S.No. | Process Step | Column Volume (CV) | Linear Flow Rate (cm/hr) |
|---|---|---|---|
| 1 | Cleaning | 10 | <300 |
| 2 | Pre Equilibration | 10 | <300 |
| 3 | Equilibration | 20 | <300 |
| 4 | Loading | As Actual | <300 |
| 5 | Post Load Wash | 20 | <300 |
| 6 | Cleaning | 10 | <300 |

### 3. Nano-filtration:

20 nm nanofilter i.e. Viresolve PRO (Merck), was used to remove any virus particles available in the therapeutic protein.

### 4. Tangential flow filtration / Ultra flow filtration:

The antibody was concentrated to desired concentration and buffer exchanged in one of the three formulation buffers.

### Material used:

### Formulation Buffer:

- Buffer 1: 25 mM Histidine buffer + 75 mM Arginine Buffer + 75 mM NaCl , pH 6.50 ± 0.25;
- Buffer 2: 25mM Histidine, 75mM Arginine, 101mM NaCl;
- Bufffer 3: 25mM Histidine, 75mM Arginine, 75mM to 101mM NaCl, Polysorbate-80 0.002 % w/v
- **Cleaning Buffer: 0.5 M NaOH**

### Storage Buffer: 0.1 M NaOH

### Membrane used: PALL Centramate T Series, PES membrane MWCO: 30 kDa

### Process Parameters:

**Table 7**

| S. No. | Process Step | Description | Remark |
|---|---|---|---|
| 1 | Cleaning | 0.5 M NaOH | 30 Minutes recirculation |
| 2 | WFI Cleaning | WFI, till conductivity comes below 1.3 µS/cm | - |
| 3 | Equilibration | 400 ml | - |
| 4 | Concentration and Diafiltration | - 10-12 DV pass | - 25 |
| 5 | WFI | WFI wash 1000 mL | - |
| 6 | Cleaning | 0.5 M NaOH | 30 Minutes recirculation |

### Sterile filtration

Stabilizer was added to the antibody solution and sterile filtered through 0.2 µ filter.

### Results:

Stage wise recovery of the various steps used in the purification process.

**Table 8:**

| **S.No.** | **Stage** | **Recovery (%)** | **Purity (%)** |
|---|---|---|---|
| 1 | Protein-A Affinity Chromatography | 98 | 99.3 |
| 2 | Low pH Viral Inactivation | 98 | - |
| 3 | Cation Exchange Chromatography | 90 | 99.52 |
| 4 | Anion Exchange Chromatography | 95 | 99.46 |
| 5 | Nanofiltration | 100 | - |
| 6 | TFF/UFF | 98 | - |
| 7 | Formulation and Sterile filtration | 100 | - |

The overall process recovery was found to be ~ 80% and overall purity was found to be >99%.

**Table 9: Impurity data**

| **Assay** | **Accepted Criterion** | **Batch 1** | **Batch 2** |
|---|---|---|---|
| **Purity by HP-SEC (% monomer)** | Monomer should be >90.00%. | | |
| | Retention time of monomer should be comparable to reference standard | 99.74 | 99.11 |
| **Residual CHO DNA (pg/mg of mAb)** | <2 pg/mg IgG | 0.005 | 0.004 |
| **Residual Protein A (ng/mg of mAb)** | ≤ 10.00 ng/mg IgG | 1.05 | 0.82 |
| **Residual CHO Protein (ng/mg of mAb)** | ≤ 100.00 ng/mg IgG | 1.74 | 3.80 |
| **Endotoxin (EU/mg of protein)** | ≤ 0.1 EU/mg of protein | <0.05 | <0.05 |

**Table 10 : Batch wise Recovery & Purity**

| S.No. | Batch No. | Recovery (%) | Purity (%) |
|---|---|---|---|
| 1 | Batch 1 | 85 | 99.21 |
| 2 | Batch 2 | 87 | 99.26 |

### Example 3:

Purified Dengue (VIS513) monoclonal antibody was formulated as follows:
Excipients i.e. Arginine, Histidine, NaCl, Sucrose, and polysorbate-80 were added and mixed thoroughly using a magnetic stirrer at 50-60 RPM to form a mixture of excipients. This mixture was then added into the Dengue mAb TFF harvest gradually with stirring rate 50-60 RPM. pH was checked (pH 6.5) and if required adjusted by histidine-arginine buffer. The final formulation was filtered through a 0.2µM filter and filled into final container.

The concentration of each component in the final formulation was as follows:

**Table 11:**

| Ingredient | Formulation 1 | Formulation 2 | Formulation 3 |
|---|---|---|---|
| Dengue Mab (VIS513) | 10 mg/ml | 25 mg/ml | 50 mg/ml |
| Histidine | 25 mM | 25 mM | 25 mM |
| Arginine | 75 mM | 75 mM | 75 mM |
| Sodium Chloride | 101mM | 101mM | 101mM |
| Sucrose | 0.5 % w/v | 0.5 % w/v | 0.5 % w/v |
| Polysorbate-80 | 0.02% w/v | 0.02% w/v | 0.02% w/v |
| | | | |
| pH | 6.5±0.5 | 6.5±0.5 | 6.5±0.5 |
| Osmolality | 380 mOsm/kg | 380 mOsm/kg | 380 mOsm/kg |
| | | | |

These formulations were further tested for purity, stability, efficacy and potency for 9 months.

### Example 4:

Effect of presence of Sucrose in VIS513 Dengue antibody formulation was studied for testing potency by ELISA assay on EDIII protein of DV1. The formulation studies were done for temp. 2 - 8 °C, 25 °C, and 40°C.

### Results :

### 1. VIS513 Dengue antibody formulation without Sucrose

**Table 12:**

| **Ingredient (QTY)** | **% Potency compared to Reference Standard Stored at 2-8°C** | | | |
|---|---|---|---|---|
| | 2-8°C | | RT (25°C) | 40°C |
| | 15 Days | 30 Days | 15 Days | 30 Days |
| L- Histidine 25mM | 78.8 | 73.2 | 79.8 | 53.8 |
| L- Arginine 75mM | | | | |
| Sodium chloride 75mM | | | | |
| Polysorbate 80 0.02% w/v | | | | |

### 2. VIS513 Dengue antibody formulation with Sucrose

**Table 13:**

| **Ingredient (QTY)** | **% Potency compared to Reference Standard Stored at 2-8°C** | | | |
|---|---|---|---|---|
| | 2-8°C | | RT (25°C) | 40°C |
| | 15 Days | 30 Days | 15 Days | 30 Days |
| L- Histidine 25mM | 90.5 | 86.3 | 94.6 | 81.6 |
| L- Arginine 75mM | | | | |
| Sodium chloride 101mM | | | | |
| Polysorbate 80 0.02% w/v | | | | |
| Sucrose 0.5% w/v | | | | |

**Reference Standard Formulation composition:**

| **Ingredient** | **(QTY)** |
|---|---|
| L- Histidine | 25mM |
| L- Arginine | 75mM |
| Sodium chloride | 101mM |
| Polysorbate 80 | 0.02% w/v |
| Sucrose | 0.5 % w/v |

Conclusion: Addition of 0.5% w/v improves stability as compared to the corresponding sampling point without sucrose.

### Example 5:

VIS513 antibody formulation was stored at 40°C for 20 days and later potency of VIS513 was evaluated by ELISA test. Effect of increasing Sucrose Strength was studied on VIS513 antibody formulation at 40°C, wherein sucrose concentration of 0.1, 0.2 and 0.5% was evaluated.

### Results:

**Table 14:**

| **Ingredient (QTY)** | **% Potency compared to Reference Standard stored at 2-8°C** |
|---|---|
| L- Histidine 25mM | 53.8 |
| L- Arginine 75mM | |
| Sodium chloride 101mM | |
| Polysorbate 80 0.02% w/v | |

**Table 15:**

| **Ingredient (QTY)** | **% Potency compared to Reference Standard stored at 2-8°C** |
|---|---|
| L- Histidine 25mM | 70.8 |
| L- Arginine 75mM | |
| Sodium chloride 101mM | |
| Polysorbate 80 0.02% w/v | |
| Sucrose 0.1% w/v | |

**Table 16:**

| **Ingredient (QTY)** | **% Potency compared to Reference Standard stored at 2-8°C** |
|---|---|
| L- Histidine 25mM | 65.7 |
| L- Arginine 75mM | |
| Sodium chloride 101mM | |
| Polysorbate 80 0.02% w/v | |
| Sucrose 0.2% w/v | |

**Table 17**

| **Ingredient (QTY)** | **% Potency compared to Reference Standard stored at 2-8°C** |
|---|---|
| L- Histidine 25mM | 81.6 |
| L- Arginine 75mM | |
| Sodium chloride 101mM | |
| Polysorbate 80 0.02% w/v | |
| Sucrose 0.5% w/v | |

**Reference Standard Formulation composition:**

| **Ingredient** | **(QTY)** |
|---|---|
| L- Histidine | 25mM |
| L- Arginine | 75mM |
| Sodium chloride | 101mM |
| Polysorbate 80 | 0.02% w/v |
| Sucrose | 0.5 % w/v |

Conclusion: Highest stability was observed formulation comprising 0.5% Sucrose.

### Example 6:

Analytical test for purity, stability, efficacy and potency of Dengue (VIS513) Mab formulation with storage at
- 2-8 °C for a period of 0 months, 3 months, 6 months, and 9 months.
- 25 °C for a period of 0 days and 30 days
- 40 °C for a period of 0 days, 7 days, 14 days, 28 days, 35 days, and 42 days.

### 6.1 : Potency of VIS513 antibody formulation was tested by indirect ELISA.

The indirect ELISA based method was used to quantify binding of Dengue Mab (VIS513) to EDIII protein of DV1 antigen. EDIII protein was immobilized to the plate. Unbound antigen was removed by washing. In the next, step standard and test samples were added, allowed to bind to the antigen. To determine the amount of bound Dv-Mab, Mouse anti-Human IgG Fc-HRP, specific to Dv-Mab (human Immunoglobulin Fc fragment), was used to recognize the presence of Dv-Mab. The assay was developed with TMB Microwell Peroxidase Substrate System which quantifies the extent of binding by amount of color formed at 450 nm. The data analysis software generated a binding curve for each sample using a four parameter curve fitting model, and compared the binding curve of the test sample to the standard curve by calculating Relative Potency. The potency of a test sample is reported as % Activity relative to reference standard (Relative Potency times 100).

### Results:

**Table 18: Dengue (VIS513) Mab potency (%) by indirect ELISA for formulation stored at 2 - 8 °C.**

| | **0 day** | **3 months** | **6 months** | **9 months** |
|---|---|---|---|---|
| **Batch 1** | 74.90 | 79.30 | 89.30 | 84.9 |
| | | | | |
| **Batch 2** | 74.30 | 80.05 | 82.20 | 86.7 |

**Table 19: Dengue (VIS513) Mab potency (%) by indirect ELISA for formulation stored at 25 °C.**

| | **0 day** | **30 days** |
|---|---|---|
| **Batch 1** | 74.90 | 79.30 |
| **Batch 2** | 74.30 | 87.8 |

**Table 20: Dengue (VIS513) Mab potency (%) by indirect ELISA for formulation stored at 40 °C.**

| | **0 day** | **14 days** | **21 days** | **28days** | **35 days** | **42 days** |
|---|---|---|---|---|---|---|
| **Batch 1** | 70.3 | 82.80 | 96.20 | 71.90 | 89.70 | 90.80 |

Dengue (VIS513) mab formulation did not show any time dependent loss of binding affinity.

### 6.2 : PRNT assay to determine EC50

The assay involves premixing serially diluted antibody with virus to allow antibody binding,-neutralization then transfer of mixture to a Vero cell monolayer, overlay with a viscous medium, incubation (~3-7 days, depending on virus serotype) to allow limited virus replication and spread, followed by detection of plaques. Neutralization was captured by the reduction of plaque formation. Robust detection was achieved with immunostaining methods, using mouse 4G2 Anti-Dengue antibody and HRP-labelled goat anti-mouse antibody with Peroxidase substrate.

The Dengue (VIS513) Mab formulation samples were been tested against all four serotypes of dengue viruses i.e. DV1, DV2, DV3 and DV4. EC50 value was calculated for neutralization of Dengue viruses. EC50 value represents the 50% effective concentration required for the effective neutralization of dengue viruses and EC50 value calculated from number of plaques present in the virus control wells and number of plaques in the wells in which mab-Virus incubated samples were added.

### Results:

**Table 21: Dengue (VIS513) mAb EC50 (ng/ml) value by PRNT assay for formulation stored at 2 - 8 °C.**

| Batch # | Dengue virus sero types | 0 day | 3 months | 6 months | 9 months |
|---|---|---|---|---|---|
| Batch 1 | DV1 | 47.15 | 49.74 | 23.88 | 24.12 |
| | DV2 | 5.9 | 8.03 | 3.58 | 3.31 |
| | DV3 | 14.38 | 14.7111 | 5.58 | 4.57 |
| | DV4 | 30.29 | 50.75 | 23.96 | 23.35 |
| | | | | | |
| Batch 2 | DV-1 | 21.03 | 21.01 | 16.12 | 29.41 |
| | DV-2 | 3.26 | 5.69 | 4.15 | 2.87 |
| | DV-3 | 13.19 | 24.14 | 6.53 | 7.05 |
| | DV-4 | 22.46 | 22.44 | 16.61 | 19.56 |

**Table 22: Dengue (VIS513) Mab EC50 (ng/ml) value by PRNT assay for formulation stored at 25 °C.**

| Batch # | Dengue virus serotypes | 0 day | 30 days |
|---|---|---|---|
| Batch 1 | DV1 | 47.15 | 29.82 |
| | DV2 | 5.9 | 5.6 |
| | DV3 | 14.38 | 9.63 |
| | DV4 | 30.29 | 35.28 |
| | | | |
| Batch 2 | DV1 | 21.03 | 28.34 |
| | DV2 | 3.26 | 5.51 |
| | DV3 | 13.19 | 9.25 |
| | DV4 | 22.46 | 30.69 |

**Table 23: Dengue (VIS513) Mab EC50 (ng/ml) value by PRNT assay for formulation stored at 40 °C.**

| Batch # | Dengue virus DV2 serotypes | Control | Test |
|---|---|---|---|
| Batch 1 | 0 day | 6.66 | - |
| | 7 days | 6.37 | 26.89 |
| | 14 days | 6.18 | 36.12 |

Dengue (VIS513) mab formulation did not show any time dependent loss of virus neutralization efficacy at 2-8°C & 25°C. VIS513 formulation even if kept at 40°C, does not lose its ability to neutralize dengue virus.

### 6.3 : Aggregation and purity analysis

A HPLC- based size exclusion chromatography (HPLC-SEC) was used to assess the aggregates in the bulk and final formulation of DV Mab. In this method a phenomenex Bio-Sec-S 3000 column was used to demonstrate the aggregates and monomer percentage of Dengue (VIS513) Mab by injecting the -50 µg of total antibody and run at a flow rate of 1 ml/minute for 35 minutes. Phosphate buffered Saline (PBS), pH 6.5 was used as mobile phase.

### Results: SEC-HPLC (Acceptance range is NLT 90%)

**Table 24: SEC-HPLC analysis of formulation stored at 2 - 8 °C**

| **Batch** | **0 Day** | **3 months** | **6 months** | **9 month** |
|---|---|---|---|---|
| 1 | 99.21 | 98.60 | 99.14 | 98.55 |
| 2 | 99.26 | 98.81 | 98.89 | 98.53 |

**Table 25: SEC-HPLC analysis of formulation stored at 25 °C**

| | **0 day** | **30 days** |
|---|---|---|
| **Batch 1** | 99.21 | 97.63 |
| **Batch 2** | 99.26 | 97.58 |

**Table 26: SEC-HPLC analysis of formulation stored at 40 °C**

| | **0 day** | **7 days** | **14 days** | **21 days** | **28days** | **35 days** | **42 days** |
|---|---|---|---|---|---|---|---|
| **Batch 1** | 99.21 | 99.30 | 99.50 | 97.74 | 97.30 | 98.70 | 95.30 |

Dengue (VIS513) mab formulation did not show any significant time dependent aggregation; and purity/ monomer content was found to be >98%.

### Example 7:

Effect of surfactant concentrations of Formulations:
Effect of Surfactant concentration was evaluated by sub-visible particle analysis. Formulations varying Polysorbate-80 strengths were prepared and analyzed for Sub visible particle analysis.

**Table 27**

| | **Formulation with Polysorbate 80 conc (% W/V)** | | |
|---|---|---|---|
| | 0.0016 % | 0.002% | 0.005% |
| **Particle size** | | | |
| ≥2 µ | 916 | 211 | 20 |
| ≥ 5µ | 55 | 48 | 5 |
| ≥10µ | 6 | 16 | 2 |
| ≥25 µ | 1 | 1 | 0 |

### Conclusion:

In the formulation containing 0.005% w/v Polysorbate-80 minimum sub visible particles were observed. Depending on dose, if the formulation requires dilution Polysorbate 80 strength was finalized 0.02% w/V with margin of 4 fold.

### Example 8: Study of determination of minimum concentration of stabilizers used

Minimum buffer strength required (10-30mM) was referred from the available literature. To find out minimum Arginine (used as solubilising agent and viscosity reducing agent) Mab sample was buffer exchanged into normal saline and Arginine stock solution (300mM) was gradually added. The aggregation of the solution was monitored by measuring OD@350nm. The saline with 75mM Arginine gave lowest OD hence 75mM Arginine was finalized.

### Example 8

### Viscosity studies of Dengue (VIS513) antibody formulation

Viscosity of DV mab samples was measured on a microchip based Viscometer, Model: microVISCTM (Make: RheoSense, CA USA) as per procedure mentioned in the instrument manual.

**Table 28**

| Sample | Measurement Temperature | Viscosity mPa-S |
|---|---|---|
| Day 0 | 5°C | 2.04 |
| | 25°C | 1.164 |
| Day 90 stored at 2-8°C | 25°C | 1.173 |
| Day 30 stored at 25°C | 25°C | 1.137 |

### Conclusion:

No time dependent increase in viscosity was observed in the mab formulation stored at 2-8°C for 90 days as well at a sample kept at 25°C for 1 month, this is primarily due to the excipient-Arginine 75mM.Viscosity of our formulation was found to be 1.1 to 1.2 mPa-S /cP, which is lower than other marketed formulations that have viscosity between 11-50 mPa-S /cP.

### Example 9: Viral Spiking Studies on Dengue (VIS513) mAb purification process

Virus validation was performed for actual manufacturing process, to test the effectiveness of the virus removal by virus filtration in the manufacturing process of monoclonal antibody.

Murine Leukemia Virus (MuLV) and Minute virus of mice (MMV/MVM) were used as model organisms. Inventors of this invention compared the ability of their inventive purification process with that of the general and well established method of monoclonal antibody purification.

The general and well established method of monoclonal antibody purification comprised of Protein-A Affinity Chromatography (GE Resin); Low pH Treatment; Sartobind Q Chromatography (Anion Exchange Membrane, Sartorius, single use); Sartobind Phenyl Chromatography (Membrane Chromatography, Sartorius, single use); Viresolve Pro filtration (Nanofiltration, Merck).

**Table 29:**

| Step | Log₁₀ Viral Reduction Factor (LRV) | |
|---|---|---|
| | MuLV | MMV |
| General /Standard method | 12.64 ± 0.60 | 7.02 ± 0.69 |
| Inventive method (SIIPL) | 23.74 ± 0.60 | 11.91 ± 0.70 |

### Results:

SIIPL purification process was highly efficient in viral clearance, total LRV achieved is as per the ICH guidelines. (Standard Process LRV 12.64 while SIIPL inventive process 23.74) Dengue antibody purified using our inventive process was found to be suitable for human clinical trials without any viral risk.

### Example 10: Upstream process for cell culturing and expression of therapeutic protein i.e. Rabies Monoclonal antibody

### Protocol:

Cell culturing at 2 L scale was carried out in a Fed batch manner using below mentioned parameters during fermentation/upstream process.
- The rabies monoclonal antibody was expressed in cell line "GS - CHO".
- Cell culture medium used for cell growth and expression of therapeutic protein i.e. Rabies monoclonal antibody was "1X Cellvento^{™} CHO-220 Liquid Medium" or "Actipro 1x (Hyclone)"
- Feed solution A, Feed solution B, Feed solution C, Feed solution D selected from group comprising of Glucose, Cell Boost^{™} 5 Supplement (Hyclone), EX-CELL 293 (Sigma Aldrich), Cell Boost 7a and 7b supplements (Hyclone), 3X Actipro (Hyclone), Cell Vento 220 (1X medium), EX-CELL^{®} Advanced^{™} CHO Feed was used for supplementation feed.
- pH of the fermentation medium was maintained at 6.5 to 7.5.
- Osmolality of the fermentation medium was maintained between 270 - 450 mOsm/Kg.
- Dissolved oxygen of the fermentation medium was maintained at about 20% to about 60%.
- Temperature of the fermentation medium was maintained at 36.5 ± 1.

Feed supplementation was done in a gradual drip manner as per following table:

**Table 30:**

| Day | Feed A | Feed B | Feed C | Feed D | Basal Medium 1 (3X Actipro" (Hyclone) | Basal Medium 2 (Cellvento^{™} CHO-220 (1X)) |
|---|---|---|---|---|---|---|
| 0 | | | | | | |
| 1 | | | | | | |
| 2 | | 4% | | | | |
| 3 | 0.2% | | | | | |
| 4 | | | 4% | 0.4% | 10% | 8% |
| 5 | | 6% | 4% | 0.4% | | |
| 6 | 0.2% | 4% | 4% | 0.4% | | |
| 7 | | 6% | | | | |
| 8 | 0.2% | | 4% | 0.4% | | |
| 9 | | 4% | 4% | 4.0% | | |
| 10 | 0.1% | 2% | 4% | 0.4% | | |
| 11 | 0.2% | 2% | 2% | 0.2% | | |
| 12 | | 4% | | | | |
| 13 | 0.1% | 2% | 2% | 0.2% | | |
| 14 | | 3% | | | | |
| 15 | | 3% | | | | |
| 16 | | | | | | |

| | | | | | | |
|---|---|---|---|---|---|---|
| Note: All feeding solutions may vary by ±1%and by ± 1 day. | | | | | | |

The cell culture was harvested upon drop in OD upto 60%

### Results & conclusion:

Yield of 3 - 5 gm/L was obtained of the fermentation process. Harvest obtained was further subjected to purification / downstream processing.

### Example 11:

Cell culture obtained according to example 9 was harvested and later subjected to protocol for purification of the rabies monoclonal antibody as per Figure 1.

Detailed process used was as follows:
Protein-A Affinity Chromatography:
In this step targeted monoclonal antibody was separated from the media components in the harvested supernatant. Clarified supernatant was passed through the chromatography column and then eluted using compatible elution buffers.

Materials used:
**Resin (Matrix):** Mab Select Sure/Eshmuno A (Protein-A Affinity)
**Residence Time:** 4.0-8.0 minutes
**Column used:** XK 26
**Equilibration Buffer:** 20 mM Phosphate Buffer + 150 mM NaCl + 0.05 %(w/v) Polysorbate 80, pH 7.0±0.2.
**Wash** I **Buffer:** 20 mM Phosphate Buffer + 150mM NaCl + 0.05% (w/v) Polysorbate 80, pH 7.0±0.2.
**Wash II Buffer:** 20 mM Phosphate Buffer + 1M NaCl + 0.05% (w/v) Polysorbate 80, pH 7.0±0.2.
**Wash III Buffer**: 10 mM Phosphate Buffer + 125 mM NaCl + 0.025% (w/v) Polysorbate 80, pH 6.0±0.2.
**Elution Buffer**: 20 mM Citrate buffer + 0.025% (w/v) Polysorbate 80, pH 3.0±0.2.
**CIP Buffer:** 0.1 M NaOH

### Process Parameters used:

**Table 31**

| S. No. | Process Step | Column Volume | Linear Flow Rate (cm/hr) |
|---|---|---|---|
| 1 | Equilibration | 5 | <300 |
| 2 | Loading (mL) | As Actual | <300 |
| 3 | Wash I | 2 | <300 |
| 4 | Wash II | 4 | <300 |
| 5 | Wash III | 4 | <300 |
| 6 | Elution | 5 | <300 |
| 7 | Cleaning | 3 | <300 |
| 8 | Storage | 2 | <300 |

### 1. Low pH Viral inactivation

i. Eluate from protein-A Affinity chromatography was subjected to low pH i.e. 3.5±0.1 for 60±10 minutes to inactivate the viral particles.
ii. After low pH hold, eluate was neutralized using neutralization buffer i.e. 1 M Tris/Citrate buffer having pH 7.0±0.2. Subsequently neutralized solution was filtered using 0.8/0.45 µ or 0.8/0.2 µ.
iii. Conductivity of neutralized eluate was adjusted using WFI with 0.025% (w/v) Polysorbate 80.

### 2. Cation Exchange Chromatography

Positively charged antibody molecules bound with the column while negatively charged molecules come in the flow through. Column bound antibody molecules are eluted using salt gradient.

### Materials used:

**Resin used:** Fractogel SO3⁻/ Fractogel SE Hicap (Merck)
**Residence Time:** 4.00-7.00 minutes
**Column used:** XK 26
**Pre Equilibration:** 200 mM Citrate buffer pH 6.0±0.2.
**Equilibration:** 10 mM Citrate buffer +0.025% (w/v) Polysorbate 80, pH 6.0±0.2.
**Loading:** Low pH Hold neutralized.
**Wash Buffer A:** 10 mM Citrate buffer, pH 6.0±0.2.
**Wash Buffer B**: 20 mM Citrate buffer + 300 mM NaCl, pH 6.0±0.2.
**CIP Buffer:** 0.5 M NaOH
**Storage Buffer:** 20% ethanol + 150mM NaCl

**Table 32: Process Parameters:**

| **S. No.** | **Process Step** | **CV** | **Linear Flow Rate (cm/hr)** |
|---|---|---|---|
| 1 | Pre Equilibration | 2 | <300 |
| 2 | Equilibration | 5 | <300 |
| 3 | Loading | As Actual | <300 |
| 4 | Wash | 5 | <300 |
| 5 | Elution 0-60% B (Gradient) | 15 | <300 |
| 6 | 100 % B | 2 | <300 |
| 7 | CIP | 3 | <300 |

**Table 33: Fraction collection during gradient**

| S.No. | Fraction Name | Collection Criteria (UV₂₈₀) mAU |
|---|---|---|
| 1 | Fraction 01 | Upto 300 |
| 2 | Fraction 02 | 300 - 700 |
| 3 | Fraction 03 | 700 till base line |
| 4 | Fraction 04 (100% B) | Baseline to base line |

### Anion Exchange Chromatography:

All negatively charged impurities are bound with the membrane while antibody comes in the flow through.

Materials used:
**Membrane/Resin used:** Sartobind Q single Sep mini (Sartorius)/Eshmuno Q
Loading volume: 150mg/mL-1000 mg/mL
Column used: XK 26
Cleaning Buffer: 0.5 M NaOH
Pre-equilibration buffer: 200 mM Citrate buffer, pH 6.0 ±0.2.
Equilibration Buffer: 20 mM Citrate buffer pH 6.0 ± 0.2 ; and optionally 0.025% PS-80 pH 6.0±0.2
**Storage Buffer:** 20% ethanol + 150mM NaCl or 0.1 M NaOH

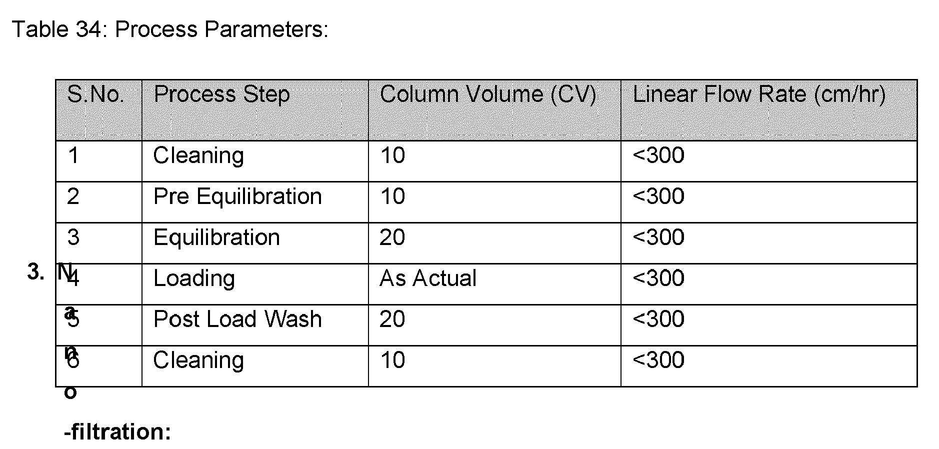

20 nm nanofilter i.e. Viresolve PRO (Merck), was used to remove any virus particles available in the therapeutic protein.

### 4. Tangential flow filtration / Ultra flow filtration:

The antibody was concentrated to desired concentration and buffer exchanged in one of the three formulation buffers.

### Material used:

### Formulation Buffer:

- Buffer 1: 25 mM Histidine buffer + 75 mM Arginine Buffer + 75 mM NaCl , pH 6.50 ± 0.25;
- Buffer 2: 25mM Histidine, 75mM Arginine, 101mM NaCl;
- Bufffer 3: 25mM Histidine, 75mM Arginine, 75mM to 101mM NaCl, Polysorbate-80 0.002 % w/v
- Cleaning Buffer: 0.5 M NaOH

### Storage Buffer: 20% ethanol + 150mM NaCl or 0.1 M NaOH

### Membrane used: PALL Centramate T Series, PES membrane MWCO: 30 kDa

**Table 35: Process Parameters:**

| S. No. | Process Step | Description | Remark |
|---|---|---|---|
| 1 | Cleaning | 0.5 M NaOH | 30 Minutes recirculation |
| 2 | WFI Cleaning | WFI, till conductivity comes below 1.3 µS/cm | - |
| 3 | Equilibration | 400 ml | - |
| 4 | Concentration and Diafiltration | - 10-12 DV pass | - |
| 5 | WFI | WFI wash 1000 mL | - |
| 6 | Cleaning | 0.5 M NaOH | 30 Minutes recirculation |

### Sterile filtration

Stabilizer was added to the antibody solution and sterile filtered through 0.2 µ filter.

### Results:

**Table 36: Stage wise recovery of the various steps used in the purification process.**

| **S.No.** | **Stage** | **Recovery (%)*** |
|---|---|---|
| 1 | Protein-A Affinity Chromatography | 94 |
| 2 | Low pH Viral Inactivation | 98 |
| 3 | Cation Exchange Chromatography | 94 |
| 4 | Anion Exchange Chromatography | 95 |
| 5 | Nanofiltration | 100 |
| 6 | TFF/UFF | 98 |
| 7 | Formulation and Sterile filtration | 100 |

The overall process recovery was found to be > 80%.

**Table 37: Impurity data**

| **Assay** | **Accepted Criterion** | **Batch 1** | **Batch 2** |
|---|---|---|---|
| **Concentration by UV280 (mg/ml)** | - | 26.19 | 24.39 |
| **Purity by SEC-HPLC (% monomer)** | Monomer should be >90.00%. | 100 | 100 |
| | Retention time of monomer should be comparable to reference standard | | |
| **SDS PAGE NR (kD)** | - | 156.0 | 156.0 |
| **SDS PAGE R (kD)** | - | 50.4/27.7 | 50.7/27.9 |
| **Residual CHO DNA (pg/mg of mAb)** | <2 pg/mg IgG | 0.34 | 0.17 |
| **Residual Protein A (ng/mg of mAb)** | ≤ 10.00 ng/mg IgG | <10 | <10 |
| **Residual CHO Protein (ng/mg of mAb)** | ≤ 100.00 ng/mg IgG | 5.50 | 7.94 |
| **Bacterial Endotoxin (EU/mg of protein)** | ≤ 0.1 EU/mg of protein | 0.35 | 0.5 |

**Table 38: Batch wise Recovery & Purity**

| S.No. | Batch No. | Recovery (%) | Purity (%) |
|---|---|---|---|
| 1 | Batch 1 | 82 | 99.4 |
| 2 | Batch 2 | 80 | 99.6 |

The overall purity of the rabies mab after purification was found to be >99% and overall recovery was found to be >80%.

### Example 12:

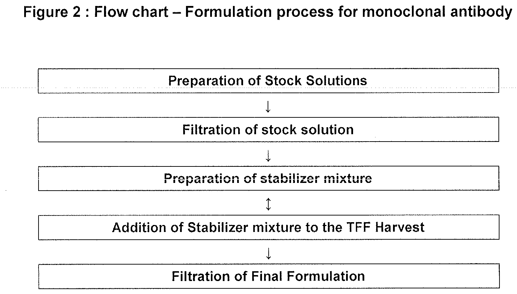

Purified Rabies monoclonal antibody was formulated as per the flowchart given in figure 2.

Excipients i.e. Arginine, Histidine, NaCl, Sucrose, and polysorbate-80 were added and mixed thoroughly using a magnetic stirrer at 50-60 RPM to form a mixture of excipients. This mixture was then added into the Dengue mAb TFF harvest gradually with stirring rate 50-60 RPM. pH was checked (pH 6.5) and if required adjusted by histidine-arginine buffer. The final formulation was filtered through a 0.2µM filter and filled into final container.

The concentration of each component in the final formulation was as follows:

**Table 39:**

| Ingredient | Formulation 1 | Formulation 2 | Formulation 3 |
|---|---|---|---|
| Rabies Mab | 10 mg/ml | 25 mg/ml | 50 mg/ml |
| Histidine | 25 mM | 25 mM | 25 mM |
| Arginine | 75 mM | 75 mM | 75 mM |
| Sodium Chloride | 101mM | 101mM | 101mM |
| Sucrose | 0.5 % w/v | 0.5 % w/v | 0.5 % w/v |
| Polysorbate-80 | 0.02% w/v | 0.02% w/v | 0.02% w/v |
| | | | |
| pH | 6.5+0.5 | 6.5+0.5 | 6.5+0.5 |
| Osmolality | 386 mOsm/kg | 386 mOsm/kg | 386 mOsm/kg |

These formulations were further tested for purity, stability, efficacy and potency for 9 months.

Example 13: Analytical test for purity & stability of Rabies Mab formulation with storage at 2-8, 25 and 40 °C for a period of 0 months, 1 month, 3 months, & 6 months.

### 13.1 Aggregation and purity analysis

A HPLC- based size exclusion chromatography (HPLC-SEC) was used to assess the aggregates in the bulk and final formulation of DV Mab. In this method a phenomenex Bio-Sec-S 3000 column was used to demonstrate the aggregates and monomer percentage of Rabies Mab by injecting the -50 ug of total antibody and run at a flow rate of 1 ml/minute for 35 minutes. Phosphate buffered Saline (PBS), pH 6.5 was used as mobile phase.

### Results:

**Table 40: SE- HPLC (%) results**

| **Temp.** | **0 day** | **1 months** | **3 months** | **6 months** |
|---|---|---|---|---|
| **2 - 8 °C** | 100 | 99.70 | 99.40 | 99.60 |
| **25 °C** | 100 | 99.60 | - | - |
| **40 °C** | 100 | 99.20 (7days) | - | - |

Rabies mab formulation did not show any time dependent aggregation and purity/ monomer content was found to be >99%.

### 13.2 SDS Page Analysis Batch 1 - Test Sample at 2-8 °C

**Table 41:**

| | | | **0 Day** | **1M** | **3M** | **6M** |
|---|---|---|---|---|---|---|
| **SDS PAGE REDUCING (kD)** | Test Sample at 2-8 °C | Heavy Chain | 50.8 | 49.8 | 49.3 | 49.2 |
| Molecular weight of heavy and light chains must be within ±10% of molecular weight of reference standard. Total % of heavy and light chain must be >95%. | | Light Chain | 25.6 | 25.7 | 25.6 | 26.4 |
| | Reference standard | Heavy Chain | 50 | 50 | 50 | 50 |
| | | Light Chain | 25 | 25 | 25 | 25 |
| Total % : 100 | | | | | | |
| | | | | | | |
| **SDS-PAGE Non-Reducing (kD)** | Test Sample at 2-8 °C | 151.0 | 156.7 | 157.2 | 157.6 | |
| **RS: Reference standard** | | | | | | |
| Molecular weight of major band must be within ± 10% of molecular weight of reference standard (RS). | Reference standard | 150 | 150 | 150 | 150 | |

### Test sample at 25 °C

| | | | **0 Day** | **30 Days** |
|---|---|---|---|---|
| **SDS PAGE REDUCING (kD)** | Test Sample at 25 °C | Heavy Chain | 50.8 | 47.5 |
| Molecular weight of heavy and light chains must be within ±10% of molecular weight of reference standard. Total % of heavy and light chain must be >95%. | | Light Chain | 25.6 | 25.5 |
| | Reference standard | Heavy Chain | 50 | 50 |
| | | Light Chain | 25 | 25 |
| Total % : 100 | | | | |
| | | | | |
| **SDS-PAGE Non-Reducing (kD) RS: Reference** | Test Sample at 25 °C | 151.0 | 151.6 | |
| **standard** Molecular | | | | |
| weight of major band must be within ± 10% of molecular weight of reference standard (RS). | Reference standard | 150 | 150 | |

**Conclusion:** Rabies mAb formulation did not show any significant time dependent changes in aggregation and change in molecular weight. Hence the formulation is found to be stable at 2 - 8 °C 25 °C, AND 40 °C.
Seq ID 1: VH amino acid sequence of VIS513 (Dengue Monoclonal Antibody) 
QVQLVQSGAEVKKPGASVKVSCKAGFNIKDVYMSWVRQAPEQGLEWMGRIDPENGDTKYD 60

PKLQGRVTMTADTSTNTAYMELRSLRSDDTAVYYCARGWEGFAYWGQGTLVTVSSASTKG 120

PSVFPLAPSSKSTSGGTAALGCLVKDYFPEPVTVSWNSGALTSGVHTFPAVLQSSGLYSL 180

SSVVTVPSSSLGTQTYICNVNHKPSNTKVDKRVEPKSCDKTHTCPPCPAPELLGGPSVFL 240

FPPKPKDTLMISRTPEVTCVVVDVSHEDPEVKFNWYVDGVEVHNAKTKPREEQYNSTYRV 300

VSVLTVLHQDWLNGKEYKCKVSNKALPAPIEKTISKAKGQPREPQVYTLPPSREEMTKNQ 360

VSLTCLVKGFYPSDIAVEWESNGQPENNYKTTPPVLDSDGSFFLYSKLTVDKSRWQQGNV 420

FSCSVMHEALHNHYTQKSLSLSPGK 445
Seq ID 2: VL amino acid sequence of VIS513 (Dengue Monoclonal Antibody) 
DIVMTQSPASLAVSLGERATISCRASENVDKYGNSFMHWYQQKPGQPPKLLIYRASELQW 60

GVPDRFSGSGSGTDFTLTISSLQAEDVAVYYCQRSNEVPWTFGQGTKLEIKRTVAAPSVF 120

IFPPSDEQLKSGTASVVCLLNNFYPREAKVQWKVDNALQSGNSQESVTEQDSKDSTYSLS 180

STLTLSKADYEKHKVYACEVTHQGLSSPVTKSFNRGEC 218
Seq ID 3: SII RmAb (RAB1)(17C7) Heavy Chain Amino Acid Sequence 
QVQLVESGGGVVQPGRSLRLSCAASGFTFSTYAMHWVRQAPGKGLEWVAVVSYDGRTKDY 60

ADSVKGRFTISRDNSKNTLYLQMNSLRTEDTAVYFCARERFSGAYFDYWGQGTLVTVSSA 120

STKGPSVFPLAPSSKSTSGGTAALGCLVKDYFPEPVTVSWNSGALTSGVHTFPAVLQSSG 180

LYSLSSVVTVPSSSLGTQTYICNVNHKPSNTKVDKRVEPKSCDKTHTCPPCPAPELLGGP 240

SVFLFPPKPKDTLMISRTPEVTCVVVDVSHEDPEVKFNWYVDGVEVHNAKTKPREEQYNS 300

TYRVVSVLTVLHQDWLNGKEYKCKVSNKALPAPIEKTISKAKGQPREPQVYTLPPSREEM 360

TKNQVSLTCLVKGFYPSDIAVEWESNGQPENNYKTTPPVLDSDGSFFLYSKLTVDKSRWQ 420

QGNVFSCSVMHEALHNHYTQKSLSLSPGK 449
Seq ID 4: SII RMAb (RAB1)(17C7) Light Chain Amino Acid Sequence

EIVLTQSPATLSLSPGERATLSCRASQSVSSYLAWYQQKPGQAPRLLIYDASNRATGIPA 60

RFSGSGSGTDFTLTISSLEPEDFAVYSCQQRNNWPPTFGGGTKVEIKRTVAAPSVSVFIF 120

PPSDEQLKSGTASVVCLLNNFYPREAKVQWKVDNALQSGNSQESVTEQDSKDSTYSLSST 180

LTLSKADYEKHKVYACEVTHQGLSSPVTKSFNRGEC 219

## Claims

1. A method of manufacturing pharmaceutical antigen binding protein with high yield and minimum aggregation, wherein the antigen binding protein is an anti-Dengue monoclonal antibody according to SEQ ID 1 and SEQ ID 2 or wherein the antigen binding protein is an anti-Rabies monoclonal antibody according to SEQ ID 3 and SEQ ID 4, the method comprising the steps of:
a) culturing in large scale mammalian cells that express antigen binding protein in a cell culture production medium, wherein the culturing step effectively maintains the cell count in the range of 10 × 10⁶ - 20 × 10⁶ cells/ml and results in a yield of at least 2 g/ L wherein the cell line of mammalian cells is CHO-K1 SV GS-KO when the antigen binding protein is an anti-Dengue monoclonal antibody and the cell line of mammalian cells is GS-CHO when the antigen binding protein is an anti-Rabies monoclonal antibody; wherein the culturing step includes use of basal medium, use of concentrated basal medium as feed solution, use of feed solutions along-with a definite feeding strategy, resulting in enhanced cell growth, maintaining lower concentrations of lactate and ammonia, and effectively maintaining the cell count thereby increasing cell longevity and high yield;
b) purification of antigen binding protein from harvested supernatant obtained in step (a), wherein the purification results in recovery of at least 80% and purity of at least 99%; and wherein the purification comprises affinity chromatography, Low pH viral inactivation, cation exchange chromatography, anion exchange chromatography, nanofiltration, Tangential flow filtration/Ultrafiltration; in a sequential manner; wherein the affinity chromatography matrix is Protein A; wherein the salt concentration of the buffers used in purification is in the range of 30 mM - 500 mM; and
c) preparing a stable formulation comprising the antigen binding protein, wherein osmolality of the formulation is in range of 300 - 400 mOsm/Kg and the viscosity of the formulation is less than 2.5 mPa-S, wherein the formulation comprises at least one antigen binding protein, at least one stabilizer, at least one buffering agent, at least one tonicity agent, and at least one surfactant;
wherein the formulation comprises:
(i) 1 - 100 mg/ml of the antigen binding protein;
(ii) 20 - 40 mM of histidine;
(iii) 50 - 100 mM of arginine;
(iv) 0.002 - 0.02% Polysorbate 80 (w/v);
(v) 50 - 150 mM NaCl; and
(vi) 0.1 - 2.5% sucrose w/v; wherein pH of the formulation is 6.5 ± 0.5;
and wherein the resulting formulation is stable at 2-8 deg C for at least 9 months, at 25 deg C for at least 1 month, at 40 deg C for at least 40 days, at 50 deg C for at least 2 days.

2. The method of claim 1, wherein the cell culture medium is supplemented with one or more other nutrients, at least once during the culturing step.

3. The method of claim 1, wherein the cell culture production medium is supplemented on a schedule comprising supplementation that is continuous, daily, every other day, every two days or a combination thereof.

4. The method of claim 1, wherein the cell culture medium has an osmolality in range of 250 - 500mOsm/Kg; pH in range of 6.5 - 7.5; dissolved oxygen is maintained in range of 10 - 60%; cell culture temperature is in the range of 30°C to 38°C; first temperature preferably 36 - 37 °C and optionally second temperature preferably 30 - 35 °C; glucose concentration is maintained below 7%; preferably between 4% and 5% ; harvesting the cell culture when viability is decreased to 80 %; wherein the cell culture conditions are maintained in a manner that secondary metabolites such as lactate concentration is not more than 5g/L; and ammonia concentration is not more than 5 mMol/L.

5. The method of claim 4, wherein the osmolality of the fermentation medium is 400 - 500 mOsm/kg.

6. The method of claim 1, wherein the dissolved oxygen of the fermentation medium is maintained in the range of 20 - 40%.

7. The method of claim 1, the cells are cultivated in a batch, fed batch, continuous mode, perfusion mode; more particularly in a fed batch mode.

8. The method of claim 1, wherein the salt concentration of the buffers used in purification is in the range of 50 mM - 300 mM.

9. The method of claim 1, further comprising an additional chromatography step selected from the group comprising one or more of Hydrophobic interaction chromatography, Hydrophobic charge induction chromatography, Ceramic hydroxyapatite chromatography, Multimodal chromatography, Membrane chromatography.

10. The method of claim 1, wherein the protein A chromatography comprises:
a) Equilibration buffer: 20 mM phosphate Buffer; 100 - 150 mM NaCl; 0.05% Polysorbate 80; pH 7.0 ± 0.2
b) Loading: Clarified Harvest
c) Wash I Buffer: 20 mM phosphate buffer; 100 - 150 mM NaCl, more particularly 150mM; 0.05% Polysorbate 80; pH 7.0 ± 0.2
d) Wash II Buffer: 20 mM phosphate buffer; 250 mM - 1 M NaCl, more particularly 1M; 0.05% Polysorbate 80; pH 7.0 ± 0.2
e) Wash III Buffer: 10 mM phosphate buffer; 100 - 150 mM NaCl, more particularly 125mM; 0.05% Polysorbate 80; pH 7.0 ± 0.2
f) Elution Buffer: 20mM citrate buffer; pH 3.0 ± 0.2; and optionally 0.025 % (w/v) Polysorbate 80
g) CIP Buffer: 0.1M NaOH
h) Residence time: 4.00 - 8.00 minutes
i) Column used: XK26
j) Linear flow rate is 10 - 500 cm/hr, more particularly 100-150 cm/hr.

11. The method of claim 1, wherein viral inactivation of the eluate from the protein A affinity chromatography is accomplished by holding the eluate at pH 3.3 - 3.5 for a period of 50 - 100 minutes.

12. The method of claim 1, wherein the cation exchange chromatography is conducted using a resin selected from the group comprising one or more of sulfonate based group; a sulfoethyl based group; a sulphopropyl based group; a sulfoisobutyl based group; a sulfoxy ethyl based group, a carboxymethyl based group; sulfonic and carboxylic acid based groups; a carboxylic acid based group; a sulfonic acid based group; and an orthophosphate based group.

13. The method of claim 1, wherein the cation exchange chromatography comprises:
a) Pre-equilibration buffer: 200 mM citrate buffer; pH 6.0 ± 0.2
b) Equilibration buffer: 10 mM citrate buffer; 0.025 % (w/v) Polysorbate 80; pH 6.0 ± 0.2
c) Wash Buffer A: 10 mM citrate buffer; pH 6.0 ± 0.2
d) Wash buffer B: 20 mM citrate buffer; 300 - 500 mM NaCl; pH 6.0 ± 0.2
e) CIP buffer: 0.5M NaOH
f) Residence time: 4.00 - 7.00 minutes
g) Column used: XK26.

14. The method of claim 1, wherein the anion exchange chromatography comprises:
a) Cleaning buffer: 0.5M NaOH
b) Pre-equilibration buffer: 200 mM citrate buffer; pH 6.0 ± 0.2
c) Equilibration buffer: 20 mM citrate buffer; pH 6.0 ± 0.2; and optionally 0.025% Polysorbate 80
d) Storage buffer: 0.1M NaOH
e) Linear Flow rate is 10 - 500 cm/hr, more particularly 100-150 cm/hr
f) Column used: XK26.

15. The method of claim 1, wherein the anion exchange chromatography is "flow through and wash mode" or "bind and elute mode".

16. The method of claim 1, wherein the removal of viral particles is accomplished by nanofiltration using virus retentive filter.

17. The method of claim 1, wherein the antigen binding protein is concentrated using Tangential Flow Filtration (TFF).

18. The method of claim 17, wherein the TFF is carried out using 30 kDa membrane.

19. The method of claim 17, wherein the Tangential Flow Filtration process comprises:
a) Diafiltration using diafiltration buffer: 25 mM histidine buffer; 75 mM arginine buffer; 50 - 150 mM NaCl; pH 6.50 ± 0.5
b) Cleaning buffer: 0.5M NaOH
c) Storage buffer: 0.1M NaOH
d) Equilibration using 5 - 10 X membrane volume
e) Concentration and diafiltration using 10-20 diafiltration volume
f) WFI wash using 3 - 5 membrane volume
g) Cleaning using 0.5 - 1.0 M NaOH
h) Storage 0.1M NaOH.

20. The method of claim 1, wherein the purified therapeutic protein preparation contains no greater than 2% aggregates, preferably less than 1% aggregates.

21. The method of claim 1, wherein the stable antigen binding protein formulation comprises 1 mg/ml to 100 mg/ml of antigen binding protein.

22. The method according to any one of the preceding claims, wherein the antigen binding protein formulation comprises of not more than 3% aggregation, minimum amount of sub-visible particles and improved potency.

23. The method of claim 1, wherein the concentration of the antigen binding protein monomer in the formulation is greater than 99%; residual CHO DNA is not more than 2 pg/mg of antigen binding protein, more particularly not more than 0.1 pg/mg of antigen binding protein; residual CHO protein is not more than 100 ng/mg of antigen binding protein, more particularly not more than 10 ng/mg of antigen binding protein; residual Protein-A is not more than 10 ng/mg of antigen binding protein, more particularly not more than 1.5 ng/mg of antigen binding protein; Endotoxin is not more than 0.1 EU/mg of antigen binding protein.

24. The method of claim 1, wherein the formulation comprises:
- <1% (w/v), most preferably 0.5% (w/v), sucrose;
- 25 mM, histidine;
- 75 mM, arginine;
- 100 - 145 mM, sodium chloride;
- 0.02%( w/v), Polysorbate 80; and
- 1 mg/ml to 50 mg/ml, antigen binding protein.

25. A pharmaceutical formulation comprising:
a) 1 - 100 mg/ml of at least one antigen binding protein, wherein the antigen binding protein is an anti-Dengue monoclonal antibody according to SEQ ID 1 and SEQ ID 2 or wherein the antigen binding protein is an anti-Rabies monoclonal antibody according to SEQ ID 3 and SEQ ID 4;
b) 20 - 40 mM of histidine;
c) 50 - 100 mM of arginine;
d) 0.002 - 0.02% Polysorbate 80 (w/v);
e) 50 - 150 mM NaCl;
f) 0.1 - 2.5% sucrose w/v; wherein pH of the formulation is 6.5 ± 0.5,
wherein the osmolality of the formulation is 300 - 450 mOsmol/kg and viscosity less than 2.5 mPa-S and said formulation is stable at 2-8 deg C for at least 9 months, at 25 deg C for at least 1 month, at 40 deg C for at least 40 days, at 50 deg C for at least 2 days.

26. A pharmaceutical formulation according to claim 25, comprising of 2 - 80 mg/ml of the least one antigen binding protein; 25 mM of histidine; 75 mM of arginine; 101 mM NaCl; 0.02% Polysorbate 80 (w/v); and 0.5% (w/v) sucrose; wherein pH of the formulation is 6.5 ± 0.5.

27. A pharmaceutical formulation according to claim 25 or 26, wherein the osmolality of the formulation is 380 mOsmol/kg.

28. A pharmaceutical formulation according to any one of claims 25 to 27, comprising 2 - 80 mg/ml antigen binding protein; 25 mM of histidine; 75 mM of arginine; 101 mM NaCl; 0.02% Polysorbate 80 (w/v); and 0.5% sucrose w/v; wherein pH of the formulation is 6.5 ± 0.5 osmolality 380 mOsm/Kg, viscosity less than 2.5 mPa-S.

29. A pharmaceutical formulation according to any one of claims 25 to 28, comprising 25 mg/ml antigen binding protein; 25 mM of histidine; 75 mM of arginine; 101 mM NaCl; 0.02% Polysorbate 80 (w/v); and 0.5% sucrose w/v; wherein pH of the formulation is 6.5 ± 0.5 osmolality 380 mOsm/Kg, viscosity less than 2.5 mPa-S.

30. A pharmaceutical formulation according to any one of claims 25 to 28, comprising 50 mg/ml antigen binding protein; 25 mM of histidine; 75 mM of arginine; 101 mM NaCl; 0.02% Polysorbate 80 (w/v); and 0.5% sucrose w/v; wherein pH of the formulation is 6.5 ± 0.5 osmolality 380 mOsm/Kg, viscosity less than 2.5 mPa-S.

31. A pharmaceutical formulation according to any one of claims 25 to 30 for use in the treatment, prevention or diagnosis of dengue or rabies virus.

32. A pharmaceutical formulation for use according to claim 31 wherein the formulation is comprised in a container selected from a bottle, a vial, an ampule, an IV bag, a wearable injector, a bolus injector, a syringe, a pen, a pump, a multidose needle syringe, a multidose pen, a injector, a syrette, an autoinjector, a pre-filled syringe, or a combination thereof.

33. A pharmaceutical formulation for use according to claim 32 wherein at least one primary packaging component comprises a container closure selected from polypropylene (PP), polyethylene terephthalate (PETG), high-density polyethylene (HDPE), polyethylene terephthalate (PET), polypentafluorostyrene (PFS), polycarbonate, polyvinyl chloride (PVC), polycyclopentane (CZ.RTM.), cyclic olefin copolymer (COC), polyolefin, and combinations or copolymers thereof.

## Patentansprüche

1. Verfahren zur Herstellung von pharmazeutischem antigenbindenden Protein mit hoher Ausbeute und minimaler Aggregation, wobei das antigenbindende Protein ein monoklonaler Anti-Dengue-Antikörper gemäß SEQ ID 1 und SEQ ID 2 ist oder wobei das antigenbindende Protein ein monoklonaler Anti-Tollwut-Antikörper gemäß SEQ ID 3 und SEQ ID 4 ist, wobei das Verfahren die Schritte umfasst:
a) Kultivieren von Säugetierzellen, die antigenbindendes Protein exprimieren, in einem Zellkulturproduktionsmedium im Großmaßstab, wobei der Kultivierungsschritt die Zellzahl im Bereich von 10 × 10⁶ - 20 × 10⁶ Zellen/ml effektiv aufrechterhält und zu einer Ausbeute von wenigstens 2 g/L führt, wobei die Zelllinie von Säugetierzellen CHO-K1 SV GS-KO ist, wenn das antigenbindende Protein ein monoklonaler Anti-Dengue-Antikörper ist, und die Zelllinie von Säugetierzellen GS-CHO ist, wenn das antigenbindende Protein ein monoklonaler Anti-Tollwut-Antikörper ist; wobei der Kultivierungsschritt die Verwendung von Grundmedium, die Verwendung von konzentriertem Grundmedium als Futterlösung, die Verwendung von Futterlösungen zusammen mit einer definierten Fütterungsstrategie, die zu erhöhtem Zellwachstum führt, niedrigere Konzentrationen an Laktat und Ammoniak aufrechterhält und die Zellzahl effektiv aufrechterhält, wodurch die Zell-Langlebigkeit und hohe Ausbeute erhöht wird;
b) Reinigen von antigenbindendem Protein aus geerntetem Überstand, erhalten in Schritt (a), wobei die Reinigung zu einer Ausbeute von wenigstens 80% und einer Reinheit von wenigstens 99% führt; und wobei die Reinigung Affinitätschromatographie, Virusinaktivierung bei niedrigem pH, Kationenaustauschchromatographie, Anionenaustauschchromatographie, Nanofiltration, Tangentialflussfiltration/Ultrafiltration umfasst; in einer sequenziellen Weise; wobei die Affinitätschromatographie-Matrix Protein A ist; wobei die Salzkonzentration der Puffer, die bei der Reinigung verwendet werden, im Bereich von 30 mM - 500 mM liegt; und
c) Herstellen einer stabilen Formulierung, die das antigenbindende Protein umfasst, wobei die Osmolalität der Formulierung im Bereich von 300 - 400 mOsm/Kg liegt und die Viskosität der Formulierung niedriger als 2,5 mPa-S ist, wobei die Formulierung wenigstens ein antigenbindendes Protein, wenigstens einen Stabilisator, wenigstens eine Puffersubstanz, wenigstens ein Tonizitätsmittel und wenigstens ein Tensid umfasst;
wobei die Formulierung umfasst:
(i) 1 - 100 mg/ml des antigenbindenden Proteins;
(ii) 20 - 40 mM Histidin;
(iii) 50 - 100 mM Arginin;
(iv) 0,002 - 0,02% Polysorbat 80 (w/v);
(v) 50 - 150 mM NaCl; und
(vi) 0,1 - 2,5% Saccharose w/v; wobei der pH der Formulierung 6,5 ± 0,5 beträgt;
und wobei die resultierende Formulierung bei 2 - 8°C für wenigstens 9 Monate, bei 25°C für wenigstens 1 Monat, bei 40°C für wenigstens 40 Tage, bei 50°C für wenigstens 2 Tage stabil ist.

2. Verfahren nach Anspruch 1, wobei das Zellkulturmedium mit einem oder mehreren weiteren Nährstoffen supplementiert wird, wenigstens einmal während des Kultivierungsschrittes.

3. Verfahren nach Anspruch 1, wobei das Zellkulturproduktionsmedium nach einem Zeitplan supplementiert wird, der eine Supplementierung umfasst, die kontinuierlich, täglich, jeden zweiten Tag, jeden dritten Tag oder eine Kombination davon ist.

4. Verfahren nach Anspruch 1, wobei das Zellkulturmedium eine Osmolalität im Bereich von 250 - 500 mOsm/Kg aufweist; einen pH im Bereich von 6,5 - 7,5; gelöster Sauerstoff im Bereich von 10 - 60% gehalten wird; die Zellkulturtemperatur im Bereich von 30°C bis 38°C liegt; die erste Temperatur vorzugsweise 36 - 37°C und fakultativ die zweite Temperatur vorzugsweise 30 - 35°C, die Glukosekonzentration unter 7% gehalten wird; vorzugsweise zwischen 4% und 5%; die Zellkultur geerntet wird, wenn die Lebensfähigkeit auf 80% gesunken ist; wobei die Zellkulturbedingungen so gehalten werden, dass sekundäre Metaboliten, wie etwa die Laktat-Konzentration, nicht höher als 5 g/L ist; und die Ammoniakkonzentration nicht größer als 5 mMol/L ist.

5. Verfahren nach Anspruch 4, wobei die Osmolalität des Fermentationsmediums 400 - 500 mOsm/kg beträgt.

6. Verfahren nach Anspruch 1, wobei der gelöste Sauerstoff im Fermentationsmedium im Bereich von 20 - 40% gehalten wird.

7. Verfahren nach Anspruch 1, wobei die Zellen in einem Batch-Modus, Fed-Batch-Modus, kontinuierlichem Modus, Perfusionsmodus kultiviert werden; insbesondere in einem Fed-Batch-Modus.

8. Verfahren nach Anspruch 1, wobei die Salzkonzentration der Puffer, die bei der Reinigung verwendet werden, im Bereich von 50 mM - 300 mM liegt.

9. Verfahren nach Anspruch 1, das weiter einen zusätzlichen Chromatographieschritt umfasst, ausgewählt aus der Gruppe, umfassend eines oder mehreres von hydrophober Wechselwirkungschromatographie, hydrophober Ladungsinduktionschromatographie, Keramikhydroxyapatitchromatographie, Multimodalchromatographie, Membranchromatographie.

10. Verfahren nach Anspruch 1, wobei die Protein-A-Chromatographie umfasst:
a) Äquilibrierungspuffer: 20 mM Phosphatpuffer; 100 - 150 mM NaCl; 0,05% Polysorbat 80; pH 7,0 ± 0,2
b) Beladung: geklärte Ernte
c) Wasch-I-Puffer: 20 mM Phosphatpuffer; 100 - 150 mM NaCl, insbesondere 150 mM; 0,05% Polysorbat 80; pH 7,0 ± 0,2
d) Wasch-II-Puffer: 20 mM Phosphatpuffer; 250 mM - 1 M NaCl, insbesondere 1 M; 0,05% Polysorbat 80; pH 7,0 ± 0,2
e) Wasch-III-Puffer: 10 mM Phosphatpuffer; 100 - 150 mM NaCl, insbesondere 125 mM; 0,05% Polysorbat 80; pH 7,0 ± 0,2
f) Elutionspuffer: 20 mM Citratpuffer; pH 3,0 ± 0,2; und fakultativ 0,025% (w/v) Polysorbat 80
g) CIP-Puffer: 0,1 M NaOH
h) Verweilzeit: 4,00 - 8,00 Minuten
i) Verwendete Säule: XK26
j) Lineare Durchflussrate beträgt 10 - 500 cm/hr, insbesondere 100 - 150 cm/hr.

11. Verfahren nach Anspruch 1, wobei die Virusinaktivierung des Eluats aus der Protein-A-Affinitätschromatographie erreicht wird durch Halten des Eluats bei pH 3,3 - 3,5 für einen Zeitraum von 50 - 100 Minuten.

12. Verfahren nach Anspruch 1, wobei die Kationenaustauschchromatographie durchgeführt wird unter Verwendung eines Harzes, das ausgewählt ist aus der Gruppe, umfassend eines oder mehrere aus einer Gruppe auf Sulfonat-Basis; einer Gruppe auf Sulfoethyl-Basis; einer Gruppe auf Sulfopropyl-Basis; einer Gruppe auf Sulfoisobutyl-Basis; einer Gruppe auf Sulfoxyethyl-Basis; einer Gruppe auf Carboxymethyl-Basis; Gruppen auf Sulfon- und Carbonsäure-Basis; einer Gruppe auf Carbonsäure-Basis; einer Gruppe auf Sulfonsäure-Basis; und einer Gruppe auf Orthophosphat-Basis.

13. Verfahren nach Anspruch 1, wobei die Kationenaustauschchromatographie umfasst:
a) Voräquilibrierungspuffer: 200 mM Citratpuffer; pH 6,0 ± 0,2
b) Äqulibrierungspuffer: 10 mM Citratpuffer, 0,025% (w/v) Polysorbat 80; pH 6,0 ± 0,2
c) Waschpuffer A: 10 mM Citratpuffer; pH 6,0 ± 0,2
d) Waschpuffer B: 20 mM Citratpuffer; 300 - 500 mM NaCl; pH 6,0 ± 0,2
e) CIP-Puffer: 0,5 M NaOH
f) Verweilzeit: 4,00 - 7,00 Minuten
g) Verwendete Säule: XK26.

14. Verfahren nach Anspruch 1, wobei die Anionenaustauschchromatographie umfasst:
a) Reinigungspuffer: 0,5 M NaOH
b) Voräquilibrierungspuffer: 200 mM Citratpuffer; pH 6,0 ± 0,2
c) Äquilibrierungspuffer: 20 mM Citratpuffer; pH 6,0 ± 0,2; und fakultativ 0,025% Polysorbat 80
d) Lagerpuffer: 0,1 M NaOH
e) Lineare Durchflussrate beträgt 10 - 500 cm/hr, insbesondere 100 - 150 cm/hr
f) Verwendete Säule: XK26.

15. Verfahren nach Anspruch 1, wobei die Anionenaustauschchromatographie "Durchfluss- und Wasch-Modus" oder "Bindungs- und Elutions-Modus" ist.

16. Verfahren nach Anspruch 1, wobei das Entfernen von Viruspartikeln durch Nanofiltration unter Verwendung eines Virusrückhaltefilters durchgeführt wird.

17. Verfahren nach Anspruch 1, wobei das antigenbindende Protein unter Verwendung von Tangentialflussfiltration (TFF) konzentriert wird.

18. Verfahren nach Anspruch 17, wobei die TFF unter Verwendung von 30 kDa-Membran durchgeführt wird.

19. Verfahren nach Anspruch 17, wobei das Tangentialflussfiltrations-Verfahren umfasst:
a) Diafiltration unter Verwendung von Diafiltrationspuffer: 25 mM Histidin-Puffer; 75 mM Arginin-Puffer; 50 -150 mM NaCl; pH 6,50 ± 0,5
b) Reinigungspuffer: 0,5 M NaOH
c) Lagerpuffer: 0,1 M NaOH
d) Äquilibrierung unter Verwendung von 5 - 10 X Membranvolumen
e) Konzentration und Diafiltration unter Verwendung von 10-20 Diafiltrationsvolumen
f) WFI-Waschung unter Verwendung von 3 - 5 Membranvolumen
g) Reinigung unter Verwendung von 0,5 - 1,0 M NaOH
h) Lagerung 0,1 M NaOH.

20. Verfahren nach Anspruch 1, wobei die gereinigte therapeutische Proteinzubereitung nicht mehr als 2% Aggregate, vorzugsweise weniger als 1% Aggregate enthält.

21. Verfahren nach Anspruch 1, wobei die stabile antigenbindende Proteinformulierung 1 mg/ml bis 100 mg/ml antigenbindendes Protein umfasst.

22. Verfahren nach eine der vorangehenden Ansprüche, wobei die antigenbindende Proteinformulierung nicht mehr als 3% Aggregation, minimale Menge an sub-sichtbaren Teilchen und verbesserte Potenz umfasst.

23. Verfahren nach Anspruch 1, wobei die Konzentration des antigenbindenden Proteinmonomers in der Formulierung höher als 99% ist; restliche CHO-DNA nicht mehr als 2 pg/mg antigenbindendes Protein, bevorzugter nicht mehr als 0,1 pg/mg antigenbindendes Protein ausmacht; restliches CHO-Protein nicht mehr als 100 ng/mg antigenbindendes Protein, bevorzugter nicht mehr als 10 ng/mg antigenbindendes Protein ausmacht; restliches Protein A nicht mehr als 10 ng/mg antigenbindendes Protein, bevorzugter nicht mehr als 1,5 ng/mg antigenbindendes Protein ausmacht; Endotoxin nicht mehr als 0,1 EU/mg antigenbindendes Protein ausmacht.

24. Verfahren nach Anspruch 1, wobei die Formulierung umfasst:
- < 1% (w/v), am bevorzugtesten 0,5% (w/v) Saccharose;
- 25 mM Histidin;
- 75 mM Arginin;
- 100 - 145 mM Natriumchlorid;
- 0,02% (w/v) Polysorbat 80; und
- 1 mg/ml bis 50 mg/ml antigenbindendes Protein.

25. Pharmazeutische Formulierung umfassend:
a) 1 - 100 mg/ml wenigstens eines antigenbindenden Proteins, wobei das antigenbindenden Protein ein monoklonaler Anti-Dengue-Antikörper gemäß SEQ IQ 1 und SEQ ID 2 ist oder wobei das antigenbindende Protein ein monoklonaler Anti-Tollwut-Antikörper gemäß SEQ ID 3 und SEQ ID 4 ist;
b) 20 - 40 mM Histidin;
c) 50 - 100 mM Arginin;
d) 0,002 - 0,02% Polysorbat 80 (w/v);
e) 50 - 150 mM NaCl;
f) 0,1 - 2,5% Saccharose w/v; wobei der pH der Formulierung 6,5 ± 0,5 beträgt,
wobei die Osmolalität der Formulierung 300 - 450 mOsmol/kg beträgt und die Viskosität weniger als 2,5 mPa-S und besagte Formulierung bei 2-8°C für wenigstens 9 Monate, bei 25°C für wenigstens 1 Monat, bei 40°C für wenigstens 40 Tage, bei 50°C für wenigstens 2 Tage stabil ist.

26. Pharmazeutische Formulierung nach Anspruch 25, umfassend 2 - 80 mg/ml des wenigstens einen antigenbindenden Proteins; 25 mM Histidin, 75 mM Arginin; 101 mM NaCl; 0,02% Polysorbat 80 (w/v); und 0,5% (w/v) Saccharose; wobei der pH der Formulierung 6,5 ± 0,5 beträgt.

27. Pharmazeutische Formulierung nach Anspruch 25 oder 26, wobei die Osmolalität der Formulierung 380 mOsmol/kg beträgt.

28. Pharmazeutische Formulierung nach einem der Ansprüche 25 bis 27, umfassend 2 - 80 mg/ml antigenbindendes Protein; 25 mM Histidin; 75 mM Arginin; 101 mM NaCl; 0,02% Polysorbat 80 (w/v); und 0,5% Saccharose w/v; wobei der pH der Formulierung 6,5 ± 0,5, die Osmolalität 380 mOsm/Kg, die Viskosität weniger als 2,5 mPa-S beträgt.

29. Pharmazeutische Formulierung nach einem der Ansprüche 25 bis 28, umfassend 25 mg/ml antigenbindendes Protein; 25 mM Histidin; 75 mM Arginin; 101 mM NaCl; 0,02% Polysorbat 80 (w/v); und 0,5% Saccharose w/v; wobei der pH der Formulierung 6,5 ± 0,5, die Osmolalität 380 mOsm/Kg, die Viskosität weniger als 2,5 mPa-S beträgt.

30. Pharmazeutische Formulierung nach einem der Ansprüche 25 bis 28, umfassend 50 mg/ml antigenbindendes Protein; 25 mM Histidin, 75 mM Arginin, 101 mM NaCl; 0,02% Polysorbat 80 (w/v); und 0,5% Saccharose w/v; wobei der pH der Formulierung 6,5 ± 0,5, die Osmolalität 380 mOsm/Kg, die Viskosität weniger als 2,5 mPa-S beträgt.

31. Pharmazeutische Formulierung nach einem der Ansprüche 25 bis 30 zur Verwendung bei der Behandlung, Prävention oder Diagnose von Dengue- oder Tollwutvirus.

32. Pharmazeutische Formulierung zur Verwendung nach Anspruch 31, wobei die Formulierung in einem Behälter enthalten ist, der ausgewählt ist aus einer Flasche, einer Phiole, einer Ampulle, einem IV-Beutel, einem tragbaren Injektor, einem Bolusinjektor, einer Spritze, einem Pen, einer Pumpe, einer Mehrfachdosis-Nadelspritze, einem Mehrfachdosis-Pen, einem Injektor, einer Syrette, einem Autoinjektor, einer vorbefüllten Spritze oder einer Kombination davon.

33. Pharmazeutische Formulierung zur Verwendung nach Anspruch 32, wobei wenigstens eine primäre Verpackungskomponente einen Behälterverschluss umfasst, ausgewählt aus Polypropylen (PP), Polyethylenterephthalat (PETG), hochdichtem Polyethylen (HDPE), Polyethylenterephthalat (PET), Polypentafluorstyrol (PFS), Polycarbonat, Polyvinylchlorid (PVC), Polycyclopentan (CZ.RTM.), cyclischem Olefin-Copolymer (COC), Polyolefin und Kombinationen oder Copolymeren davon.

## Revendications

1. Procédé de fabrication de protéine de liaison à un antigène à usage pharmaceutique avec un rendement élevé et une agrégation minimale, dans lequel la protéine de liaison à un antigène est un anticorps monoclonal anti-Dengue selon SEQ ID 1 et SEQ ID 2 ou dans lequel la protéine de liaison à un antigène est un anticorps monoclonal antirabique selon SEQ ID 3 et SEQ ID 4, le procédé comprenant les étapes de :
a) la mise en culture à grande échelle de cellules de mammifère qui expriment une protéine de liaison à un antigène dans un milieu de production de culture cellulaire, dans lequel l'étape de mise en culture maintient efficacement le nombre de cellules dans la plage de 10 × 10⁶ à 20 × 10⁶ cellules/ml et permet d'obtenir un rendement d'au moins 2 g/L, dans lequel la lignée cellulaire de cellules de mammifère est CHO-K1 SV GS-KO lorsque la protéine de liaison à un antigène est un anticorps monoclonal anti-Dengue et la lignée cellulaire de cellules de mammifère est GS-CHO lorsque la protéine de liaison à un antigène est un anticorps monoclonal antirabique ; dans lequel l'étape de mise en culture comporte l'utilisation de milieu de base, l'utilisation de milieu de base concentré en tant que solution d'alimentation, l'utilisation de solutions d'alimentation conjointement avec une stratégie d'alimentation définie, ce qui permet d'obtenir une croissance cellulaire renforcée, de maintenir des concentrations inférieures de lactate et d'ammoniac, et de maintenir efficacement le nombre de cellules ce qui augmente la longévité cellulaire et un rendement élevé ;
b) la purification d'une protéine de liaison à un antigène à partir d'un surnageant récolté obtenu à l'étape (a), dans lequel la purification permet d'obtenir une récupération d'au moins 80 % et une pureté d'au moins 99 % ; et dans lequel la purification comprend une chromatographie d'affinité, une inactivation virale à pH faible, une chromatographie d'échange de cations, une chromatographie d'échange d'anions, une nanofiltration, une filtration à courant tangentiel/ultrafiltration ; d'une manière séquentielle ; dans lequel la matrice de chromatographie d'affinité est une protéine A ; dans lequel la concentration en sel des tampons utilisés lors de la purification est dans la plage de 30 mM à 500 mM ; et
c) la préparation d'une formulation stable comprenant la protéine de liaison à un antigène, dans lequel l'osmolalité de la formulation est dans la plage de 300 à 400 mOsm/kg et la viscosité de la formulation est inférieure à 2,5 mPa-S, dans lequel la formulation comprend au moins une protéine de liaison à un antigène, au moins un stabilisant, au moins un agent de tamponnage, au moins un agent de tonicité et au moins un tensioactif ;
dans lequel la formulation comprend :
(i) de 1 à 100 mg/ml de la protéine de liaison à un antigène ;
(ii) de 20 à 40 mM d'histidine ;
(iii) de 50 à 100 mM d'arginine ;
(iv) de 0,002 à 0,02 % de Polysorbate 80 (p/v) ;
(v) de 50 à 150 mM de NaCl ; et
(vi) de 0,1 à 2,5 % de saccharose p/v ; dans lequel le pH de la formulation est de 6,5 ± 0,5 ;
et dans lequel la formulation résultante est stable de 2 à 8 degrés C pendant au moins 9 mois, à 25 degrés C pendant au moins 1 mois, à 40 degrés C pendant au moins 40 jours, à 50 degrés C pendant au moins 2 jours.

2. Procédé selon la revendication 1, dans lequel le milieu de culture cellulaire est complété avec un ou plusieurs autres nutriments, au moins une fois au cours de l'étape de mise en culture.

3. Procédé selon la revendication 1, dans lequel le milieu de production de culture cellulaire est complété selon un programme comprenant un apport complémentaire qui est continu, quotidien, un jour sur deux, tous les deux jours ou une combinaison de ceux-ci.

4. Procédé selon la revendication 1, dans lequel le milieu de culture cellulaire possède une osmolalité dans la plage de 250 à 500 mOsm/kg ; un pH dans la plage de 6,5 à 7,5 ; l'oxygène dissous est maintenu dans une plage de 10 à 60 % ; la température de culture cellulaire est dans la plage de 30 °C à 38 °C ; une première température de préférence de 36 à 37 °C et facultativement une seconde température de préférence de 30 à 35 °C ; la concentration en glucose est maintenue au-dessous de 7 % ; de préférence entre 4 % et 5 % ; la récolte de la culture cellulaire est réalisée lorsque la viabilité est diminuée jusqu'à 80 % ; dans lequel les conditions de culture cellulaire sont maintenues d'une manière telle que des métabolites secondaires tels que la concentration en lactate ne soit pas supérieure à 5 g/L ; et que la concentration en ammoniac ne soit pas supérieure à 5 mMol/L.

5. Procédé selon la revendication 4, dans lequel l'osmolalité du milieu de fermentation est de 400 à 500 mOsm/kg.

6. Procédé selon la revendication 1, dans lequel l'oxygène dissous du milieu de fermentation est maintenu dans la plage de 20 à 40 %.

7. Procédé selon la revendication 1, les cellules sont cultivées en discontinu, en semi-discontinu, en mode continu, en mode perfusion ; plus particulièrement en mode semi-discontinu.

8. Procédé selon la revendication 1, dans lequel la concentration en sel des tampons utilisés lors de la purification est dans la plage de 50 mM à 300 mM.

9. Procédé selon la revendication 1, comprenant en outre une étape de chromatographie supplémentaire sélectionnée parmi le groupe comprenant l'une ou plusieurs parmi une chromatographie d'interaction hydrophobe, une chromatographie d'induction de charge hydrophobe, une chromatographie sur céramique d'hydroxyapatite, une chromatographie multimodale, une chromatographie sur membrane.

10. Procédé selon la revendication 1, dans lequel la chromatographie sur protéine A comprend :
a) Tampon d'équilibrage : tampon phosphate à 20 mM ; de 100 à 150 mM de NaCl ; 0,05 % de Polysorbate 80 ; pH 7,0 ± 0,2
b) Chargement : récolte clarifiée
c) Tampon de lavage I : tampon phosphate à 20 mM ; de 100 à 150 mM de NaCl, plus particulièrement 150 mM ; 0,05 % de Polysorbate 80 ; pH 7,0 ± 0,2
d) Tampon de lavage II : tampon phosphate à 20 mM ; de 250 mM à 1M de NaCl, plus particulièrement 1M ; 0, 05 % de Polysorbate 80 ; pH 7,0 ± 0,2
e) Tampon de lavage III : tampon phosphate à 10 mM ; de 100 mM à 150 mM de NaCl, plus particulièrement 125 mM ; 0,05 % de Polysorbate 80 ; pH 7,0 ± 0,2
f) Tampon d'élution : tampon citrate à 20 mM ; pH 3,0 ± 0,2 ; et facultativement 0,025 % (p/v) de Polysorbate 80
g) Tampon de CIP : 0,1 M de NaOH
h) Temps de séjour : de 4,00 à 8,00 minutes
i) Colonne utilisée : XK26
j) Le débit linéaire est de 10 à 500 cm/h, plus particulièrement de 100 à 150 cm/h.

11. Procédé selon la revendication 1, dans lequel une inactivation virale du produit élué provenant de la chromatographie d'affinité sur protéine A est accomplie par le maintien du produit élué à pH 3,3 à 3,5 pendant une période de 50 à 100 minutes.

12. Procédé selon la revendication 1, dans lequel la chromatographie d'échange de cations est effectuée à l'aide d'une résine sélectionnée parmi le groupe comprenant l'un ou plusieurs parmi un groupe à base de sulfonate ; un groupe à base de sulfoéthyle ; un groupe à base de sulfopropyle ; un groupe à base de sulfoisobutyle ; un groupe à base de sulfoxyéthyle, un groupe à base de carboxyméthyle ; des groupes à base d'acide sulfonique et carboxylique ; un groupe à base d'acide carboxylique ; un groupe à base d'acide sulfonique ; et un groupe à base d'orthophosphate.

13. Procédé selon la revendication 1, dans lequel la chromatographie d'échange de cations comprend :
a) Tampon de pré-équilibrage : tampon citrate à 200 mM ; pH 6,0 ± 0,2
b) Tampon d'équilibrage : tampon citrate à 10 mM ; 0,025 % (p/v) de Polysorbate 80 ; pH 6,0 ± 0,2
c) Tampon de lavage A : tampon citrate à 10 mM ; pH 6,0 ± 0,2
d) Tampon de lavage B : tampon citrate à 20 mM ; de 300 à 500 mM de NaCl ; pH 6,0 ± 0,2
e) Tampon de CIP : 0,5 M de NaOH
f) Temps de séjour : de 4,00 à 7,00 minutes
g) Colonne utilisée : XK26.

14. Procédé selon la revendication 1, dans lequel la chromatographie d'échange d'anions comprend :
a) Tampon de nettoyage : 0,5 M de NaOH
b) Tampon de pré-équilibrage : tampon citrate à 200 mM ; pH 6,0 ± 0,2
c) Tampon d'équilibrage : tampon citrate à 20 mM ; pH 6,0 ± 0,2 ; et facultativement 0,025 % de Polysorbate 80
d) Tampon de stockage : 0,1 M de NaOH
e) Le débit linéaire est de 10 à 500 cm/h, plus particulièrement de 100 à 150 cm/h
g) Colonne utilisée : XK26.

15. Procédé selon la revendication 1, dans lequel la chromatographie d'échange d'anions est en « mode circuler et laver » ou en « mode lier et éluer ».

16. Procédé selon la revendication 1, dans lequel l'élimination de particules virales est accomplie par une nanofiltration à l'aide d'un filtre de rétention de virus.

17. Procédé selon la revendication 1, dans lequel la protéine de liaison à un antigène est concentrée à l'aide d'une filtration à courant tangentiel (TFF).

18. Procédé selon la revendication 17, dans lequel la TFF est effectuée à l'aide d'une membrane de 30 kDa.

19. Procédé selon la revendication 17, dans lequel le processus de filtration à courant tangentiel comprend :
a) Diafiltration à l'aide d'un tampon de diafiltration : tampon d'histidine à 25 mM ; tampon d'arginine à 75 mM; de 50 à 150 mM de NaCl; pH 6,50 ± 0,5
b) Tampon de nettoyage : 0,5 M de NaOH
c) Tampon de stockage : 0,1 M de NaOH
d) Équilibrage à l'aide de 5 à 10 fois le volume de membrane
e) Concentration et diafiltration à l'aide de 10 à 20 fois le volume de diafiltration
f) Lavage WFI, à l'aide de 3 à 5 fois le volume de membrane
g) Nettoyage à l'aide de 0,5 à 1,0 M de NaOH
h) Stockage à 0,1 M de NaOH.

20. Procédé selon la revendication 1, dans lequel la préparation de protéine thérapeutique purifiée ne contient pas plus de 2 % d'agrégats, de préférence moins de 1 % d'agrégats.

21. Procédé selon la revendication 1, dans lequel la formulation stable de protéine de liaison à un antigène comprend de 1 mg/ml à 100 mg/ml de protéine de liaison à un antigène.

22. Procédé selon l'une quelconque des revendications précédentes, dans lequel la formulation de protéine de liaison à un antigène ne comprend pas plus de 3 % d'agrégation, une quantité minimale de particules non-visibles et un potentiel amélioré.

23. Procédé selon la revendication 1, dans lequel la concentration du monomère de protéine de liaison à un antigène dans la formulation est supérieure à 99 % ; l'ADN CHO résiduel ne constitue pas plus de 2 pg/mg de protéine de liaison à un antigène, plus particulièrement pas plus de 0,1 pg/mg de protéine de liaison à un antigène ; la protéine CHO résiduelle ne constitue pas plus de 100 ng/mg de protéine de liaison à un antigène, plus particulièrement pas plus de 10 ng/mg de protéine de liaison à un antigène ; la protéine A résiduelle ne constitue pas plus de 10 ng/mg de protéine de liaison à un antigène, plus particulièrement pas plus de 1,5 ng/mg de protéine de liaison à un antigène ; une endotoxine ne constitue pas plus de 0,1 EU/mg de protéine de liaison à un antigène.

24. Procédé selon la revendication 1, dans lequel la formulation comprend :
- < 1 % (p/v), idéalement 0,5 % (p/v), de saccharose ;
- 25 mM d'histidine ;
- 75 mM d'arginine ;
- de 100 à 145 mM de chlorure de sodium ;
- 0,02 % (p/v) de Polysorbate 80 ; et
- de 1 mg/ml à 50 mg/ml de protéine de liaison à un antigène.

25. Formulation pharmaceutique comprenant :
a) de 1 à 100 mg/ml d'au moins une protéine de liaison à un antigène, dans laquelle la protéine de liaison à un antigène est un anticorps monoclonal anti-Dengue selon SEQ ID 1 et SEQ ID 2 ou dans laquelle la protéine de liaison à un antigène est un anticorps monoclonal antirabique selon SEQ ID 3 et SEQ ID 4 ;
b) de 20 à 40 mM d'histidine ;
c) de 50 à 100 mM d'arginine ;
d) de 0,002 à 0,02 % de Polysorbate 80 (p/v) ;
e) de 50 à 150 mM de NaCl ;
f) de 0,1 à 2,5 % de saccharose p/v ; dans laquelle le pH de la formulation est de 6,5 ± 0,5,
dans laquelle l'osmolalité de la formulation est de 300 à 450 mOsmol/kg et la viscosité inférieure à 2,5 mPa-S et ladite formulation est stable de 2 à 8 degrés C pendant au moins 9 mois, à 25 degrés C pendant au moins 1 mois, à 40 degrés C pendant au moins 40 jours, à 50 degrés C pendant au moins 2 jours.

26. Formulation pharmaceutique selon la revendication 25, comprenant de 2 à 80 mg/ml de l'au moins une protéine de liaison à un antigène ; 25 mM d'histidine ; 75 mM d'arginine ; 101 mM de NaCl ; 0,02 % de Polysorbate 80 (p/v) ; et 0,5 % (p/v) de saccharose ; dans laquelle le pH de la formulation est de 6,5 ± 0,5.

27. Formulation pharmaceutique selon la revendication 25 ou 26, dans laquelle l'osmolalité de la formulation est de 380 mOsmol/kg.

28. Formulation pharmaceutique selon l'une quelconque des revendications 25 à 27, comprenant de 2 à 80 mg/ml de protéine de liaison à un antigène ; 25 mM d'histidine ; 75 mM d'arginine ; 101 mM de NaCl ; 0,02 % de Polysorbate 80 (p/v) ; et 0,5 % de saccharose p/v ; dans laquelle le pH de la formulation est de 6,5 ± 0,5, l'osmolalité de 380 mOsm/kg, la viscosité inférieure à 2,5 mPa-S.

29. Formulation pharmaceutique selon l'une quelconque des revendications 25 à 28, comprenant 25 mg/ml de protéine de liaison à un antigène ; 25 mM d'histidine ; 75 mM d'arginine ; 101 mM de NaCl ; 0,02 % de Polysorbate 80 (p/v) ; et 0,5 % de saccharose p/v ; dans laquelle le pH de la formulation est de 6,5 ± 0,5, l'osmolalité de 380 mOsm/kg, la viscosité inférieure à 2,5 mPa-S.

30. Formulation pharmaceutique selon l'une quelconque des revendications 25 à 28, comprenant 50 mg/ml de protéine de liaison à un antigène ; 25 mM d'histidine ; 75 mM d'arginine ; 101 mM de NaCl ; 0,02 % de Polysorbate 80 (p/v) ; et 0,5 % de saccharose p/v ; dans laquelle le pH de la formulation est de 6,5 ± 0,5, l'osmolalité de 380 mOsm/kg, la viscosité inférieure à 2,5 mPa-S.

31. Formulation pharmaceutique selon l'une quelconque des revendications 25 à 30 pour une utilisation dans le traitement, la prévention ou le diagnostic du virus de la dengue ou de la rage.

32. Formulation pharmaceutique pour une utilisation selon la revendication 31 dans laquelle la formulation est comprise dans un contenant sélectionné parmi une bouteille, un flacon, une ampoule, une poche IV, un injecteur pouvant être porté, un injecteur bolus, une seringue, un stylo, une pompe, une seringue à aiguille à doses multiples, un stylo à doses multiples, un injecteur, une syrette, un auto-injecteur, une seringue préremplie ou une combinaison de ceux-ci.

33. Formulation pharmaceutique pour une utilisation selon la revendication 32 dans laquelle au moins un constituant d'emballage primaire comprend une enveloppe de contenant sélectionnée parmi le polypropylène (PP), le poly(téréphtalate d'éthylène) (PETG), le polyéthylène haute densité (HDPE), le poly(téréphtalate d'éthylène) (PET), le polypentafluorostyrène (PFS), le polycarbonate, le poly(chlorure de vinyle) (PVC), le polycyclopentane (CZ.RTM.), un copolymère d'oléfine cyclique (COC), une polyoléfine, et les combinaisons ou copolymères de ceux-ci.
